(19) Europäisches Patentamt
European Patent Office
Office européen des brevets

(11) **EP 3 702 785 A1**

(12) **EUROPEAN PATENT APPLICATION**

(43) Date of publication:
**02.09.2020 Bulletin 2020/36**

(51) Int Cl.:
**G01N 33/574** (2006.01)   **G01N 21/00** (2006.01)

(21) Application number: **20156084.4**

(22) Date of filing: **23.12.2014**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.12.2013   US 201361921694 P**

(62) Document number(s) of the earlier application(s) in accordance with Art. 76 EPC:
**14876751.0 / 3 090 264**

(71) Applicants:
• **The Scripps Research Institute**
**La Jolla, CA 92037 (US)**
• **The Board of Trustees of the Leland Stanford Junior University**
**Stanford, CA 94305-2038 (US)**

(72) Inventors:
• **KUHN, Peter**
**Solana Beach, CA 92075 (US)**

• **CARLSSON, Anders**
**226 51 Lund (SE)**
• **KOLATKAR, Anand**
**Los Angeles, CA 90007 (US)**
• **GAMBHIR, Savjiv Sam**
**Portola Valley, CA 94028 (US)**
• **NAIR, Viswam S.**
**Menlo Park, CA 94025 (US)**

(74) Representative: **Jones Day**
**Rechtsanwälte, Attorneys-at-Law, Patentanwälte**
**Prinzregentenstrasse 11**
**80538 München (DE)**

Remarks:
•This application was filed on 07.02.2020 as a divisional application to the application mentioned under INID code 62.
•Claims filed after the date of filing of the application / after the date of receipt of the divisional application (Rule 68(4) EPC).

(54) **CIRCULATING TUMOR CELL DIAGNOSTICS FOR LUNG CANCER**

(57) The invention provides a method for diagnosing lung cancer in a subject, the method comprising: (a) generating circulating tumor cell (CTC) data from a blood sample obtained from the subject based on a direct analysis of CTCs detected in the context of surrounding nucleated cells present in the sample in the absence of enrichment of the CTCs prior to detection, comprising immunofluorescent staining and morphological characteristics of nucleated cells in said sample, wherein CTCs are identified in the context of surrounding nucleated cells based on a combination of said immunofluorescent staining and morphological characteristics; (b) obtaining clinical data for said subject; and (c) combining said CTC data with said clinical data to diagnose lung cancer in said subject.

EP 3 702 785 A1

**Description**

This application claims the benefit of priority priority of U.S. provisional application serial No. 61/921,694, filed December 30, 2013, the entire contents of which are incorporated herein by reference.

The invention relates generally to the field of cancer diagnostics and, more specifically to methods for diagnosing lung cancer.

**BACKGROUND**

Cancer of the lung is the leading cause of cancer death in both women and men in the United States. In the year 2012, approximately 226,160 new cases of lung cancer were diagnosed in the US, with 164,770 deaths. The five-year survival rate for lung cancer is approximately 16%. This sobering outlook is due primarily to the fact that most patients have advanced disease at the time of presentation. Non-small cell lung cancer (NSCLC) accounts for approximately 85% of lung cancer diagnoses. For this subset of patients, surgery is often curative if presentation is early (stages I and II). Unfortunately, only approximately 30% of diagnoses fall within this early-stage category. Nearly a quarter of the US population actively smokes, and at the same time contributes to the downstream effects of secondhand smoke. While prevention remains the most important strategy to stem the epidemic of lung cancer, until that goal is realized there remains an urgent need for new and improved means of early diagnosis.

The National Lung Screening Trial (NLST) has definitively established a role for computerized tomography (CT) screening patients at high risk of lung cancer, namely current or recent smokers with a pack-year history of 30 years or more who were older than 55. Despite the mortality benefit, though, there remain concerns about the cost of delivering screening care, as well as the unnecessary procedures resulting from a 96% false positive rate. Moreover, even those patients who are followed for a concerning nodule are imperfectly diagnosed despite existing prediction models of risk. Biomarkers are therefore crucial for further stratifying patients who may benefit from treatments with curative intent rather than watchful waiting.

Although many non-invasive, blood biomarkers have been touted as clinically relevant, few have entered the clinic. Cancer biomarkers that have been in clinical use for decades such as Carcinoembryonic Antigen (CEA) and Alphafetoprotein (AFP) remain the sub-optimal standard of care despite a concerted effort to bring new technologies to the clinic. Modern, high-dimensional genomic and protein assays are limited by technical and statistical variation involved in assaying thousands of features from high dimensional datasets like proteins and microRNAs. Analyzing simpler features that are subject to less technical variation is appealing, and this could potentially bring meaningful biomarkers to the clinic with more rapidity.

Circulating tumor cells (CTCs) represent one potential advance made even more attractive by their non-invasive measurement. Cristofanilli et al., N Engl J Med 351:781-91, (2004) CTCs have been reported in the literature for over a century, primarily as pathologic research curiosities, but in 2004 the Food and Drug Administration approved the use of CTC enumeration by employing an immunomagnetic based antibody capture platform via Epithelial Cell Adhesion Molecule detection (EpCAM) (CellSearch, ™ Veridex, Raritan NJ) for monitoring response to therapy in advanced cancers. CellSearch™ was the first technology to demonstrate clinical utility by standardizing the CTC platform, and prospective, observational data have confirmed that CTC burden is related to therapeutic response and prognosis in multiple types of late-stage cancers. CTC detection in early-stage disease using CellSearch, ™ however, has been less promising due to poor detection sensitivity.

Other more technically sensitive types of platforms exist that enrich CTC populations by both EpCAM dependent and EpCAM independent techniques, with the ability to detect a 2 to 3 log-fold increase in CTCs for non-metastatic cancers. To date, CTC assays have not been well studied for risk stratifying lung nodules to determine whether CTCs could be helpful diagnostic adjuncts.

A need exists for accurate and non-invasive diagnostic methods of diagnosing patients at high risk of lung cancer. The present invention addresses this need by adding CTC data to existing clinical and imaging information to enhance diagnostic accuracy for patients undergoing evaluation for lung cancer. Related advantages are provided as well.

**SUMMARY OF THE INVENTION**

The present invention provides methods for diagnosing lung cancer in a subject comprising (a) generating circulating tumor cell (CTC) data from a blood sample obtained from the subject based on a direct analysis comprising immunofluorescent staining and morphological characteristics of nucleated cells in said sample, wherein CTCs are identified in context of surrounding nucleated cells based on a combination of said immunofluorescent staining and morphological characteristics; (b) obtaining clinical data for said subject; (c) combining said CTC data with said clinical data to diagnose lung cancer in said subject.

In some embodiments, the clinical data comprises one or more pieces of imaging data. In further embodiments, the clinical data comprises one or more individual risk factors. In some embodiments, the lung cancer is non-small cell lung cancer (NSCLC). In some embodiments, the lung cancer is early stage lung cancer. In some embodiments, the lung cancer is Stage I lung cancer. In additional embodiments, the subject is a high risk subject for non-small cell lung cancer (NSCLC).

In additional embodiments, the CTC data is generated by fluorescent scanning microscopy. In further embodiments, the methods comprise immunofluorescent staining of nucleated cells with pan cytokeratin, cluster of differentiation (CD)45 and diamidino-2-phenylindole (DAPI). In additional embodiments, the CTCs comprise distinct immunofluorescent staining from surrounding nucleated cells. In further embodiments, the CTCs comprise distinct morphological characteristics compared to surrounding nucleated cells. In some embodiments, the diagnosis is expressed as a risk score.

Other features and advantages of the invention will be apparent from the detailed description, and from the claims.

## BRIEF DESCRIPTION OF THE DRAWINGS

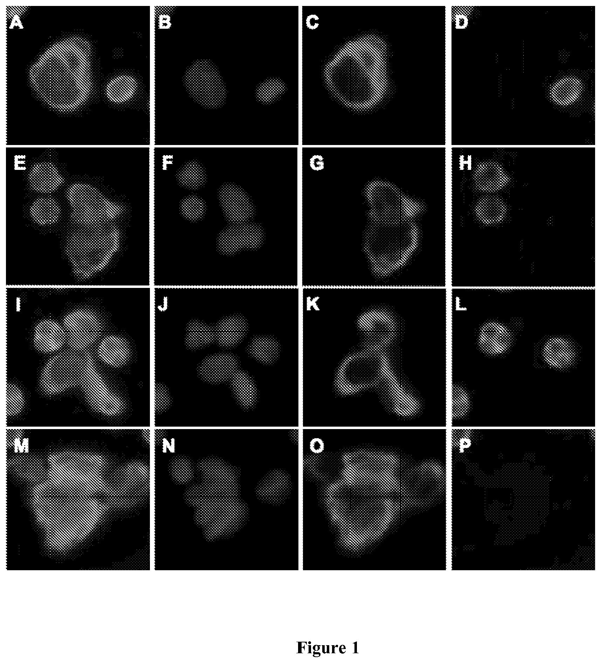

Figure 1. HD-CTCs and Tumor Clusters used for Modeling. Panel (A) shows the composite image for an HD-CTC from a patient with stage I adenocarcinoma followed by the individual DAPI positive (blue, B), Cytokeratin positive (red, C), and CD45 negative (green, D) channels defining the HD-CTC. An HD-CTC doublet (Panels E-H), triplet (Panels I-L) and "mega" cluster of more than 8 HD-CTCs (Panels M-P) are shown as composites and by individual channels. Clusters were defined as more than one CTC with touching cytoplasm (see methods) for further modeling.

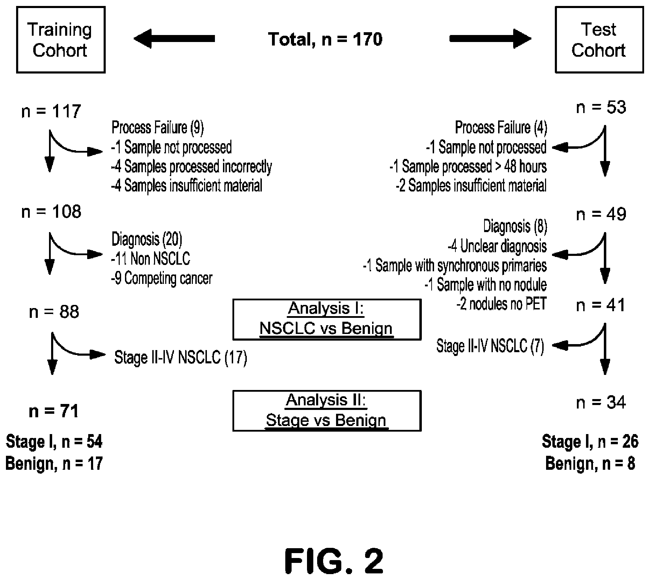

Figure 2. Patient Flow. This was a prospective, observational study of patients undergoing evaluation for a concerning lung nodule or staging for non-small cell lung cancer (NSCLC). Exclusion of patients due to blood processing errors, lack of a clear diagnosis or a non-NSCLC diagnosis, or competing cancers yielded the first analysis group of NSCLC and benign patients followed by a second analysis of the stage I only subgroup vs. benign patients only. Clinical, imaging and HD-CTC variables of interest were explored in a training set (n=88) and validated in a test set (n=41).

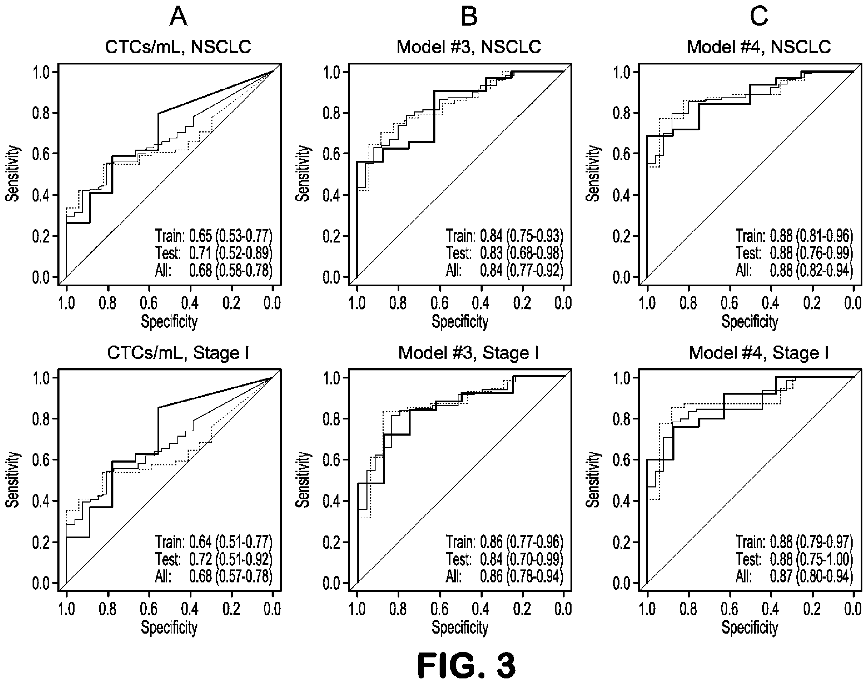

Figure 3. Receiver Operating Characteristic (ROC) Curves for HD-CTCs Only and Integrated with Clinical and Imaging Data. Receiver operating characteristic (ROC) curves for models incorporating HD-CTCs alone (A), a threshold of 7.5 HD-CTCs/mL (B) and HD-CTC clusters (present or absent) (C) for all NSCLC patients and by stage I disease only across training (dashed grey line), test (solid black line) and all (solid grey line) groups are shown. AUCs for each cohort are shown in the lower right corner of each graph with 95% confidence intervals. HD-CTCs on their own were not highly discriminating for cancer, but in combination with clinical and imaging data, a strong signal was observed in both NSCLC and stage I patients compared to benign lesions.

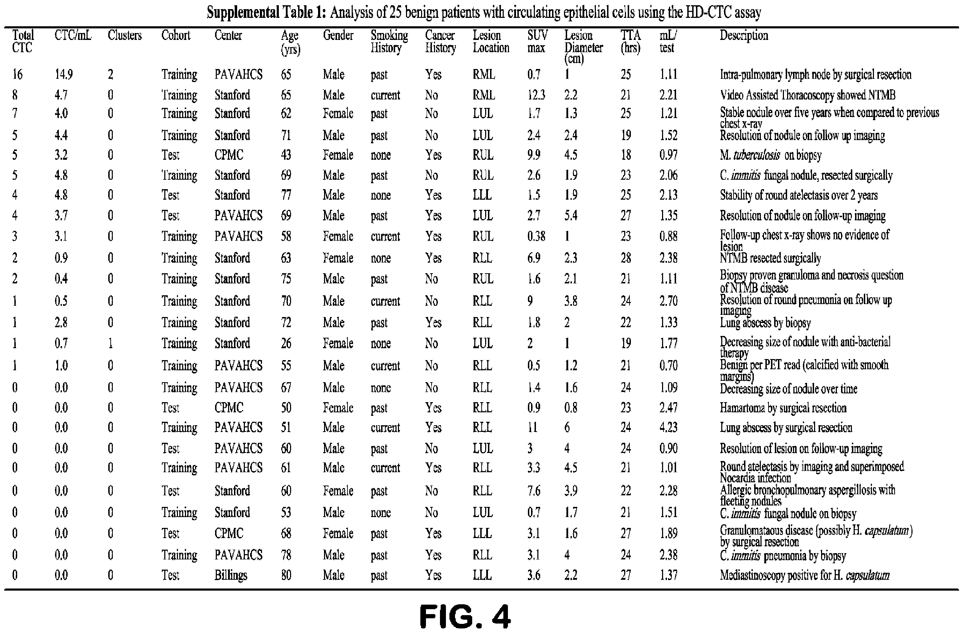

Figure 4. Supplemental Table 1. Analysis of 25 benign patients with circulating epithelial cells using the HD-CTC assay

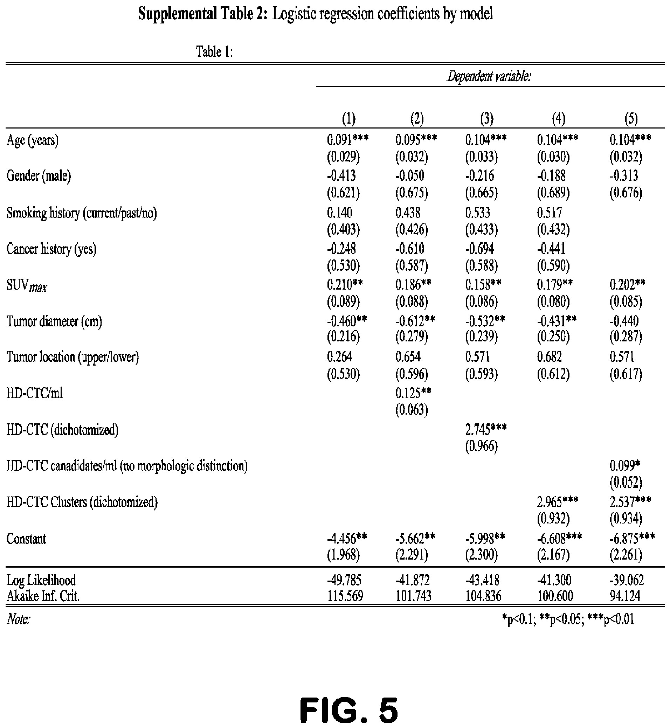

Figure 5. Supplemental Table 2. Logistic regression coefficients by model. For the risk score calculation, variables were defined as follows: Age: years alive; Gender: male = 1, female = 0; Smoking history: none=0, past = 1, current = 2; Cancer history: none = 0, yes = 1; Diameter: size in centimeters at maximal diameter; Tumor Location: lower lobe = 0, upper lobe = 1; HD-CTC clusters: none = 0; any = 1.

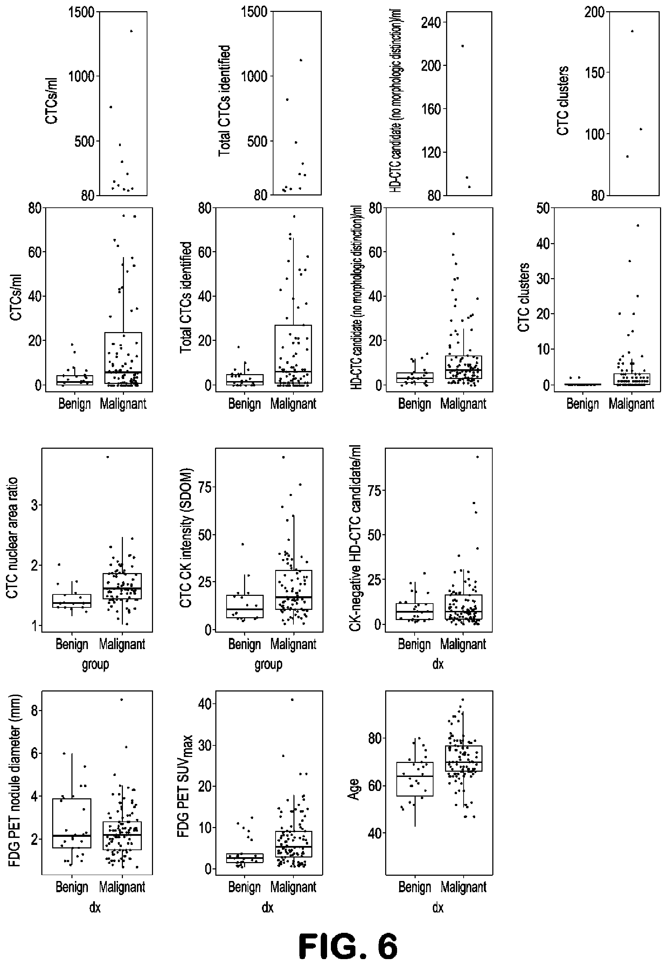

Figure 6. Variables assessed by disease group. Clinical (Age), imaging (Nodule Diameter and SUVmax) and HD-CTC variables (CTC concentration and Total CTCs, Tumor clusters, CTC size [small cells, or "SHCs," and nuclear area] and fluorescence intensity [CK intensity and CK negative cells, "DHCs"]) are shown by benign (n=25) or NSCLC diagnosis (n=104).

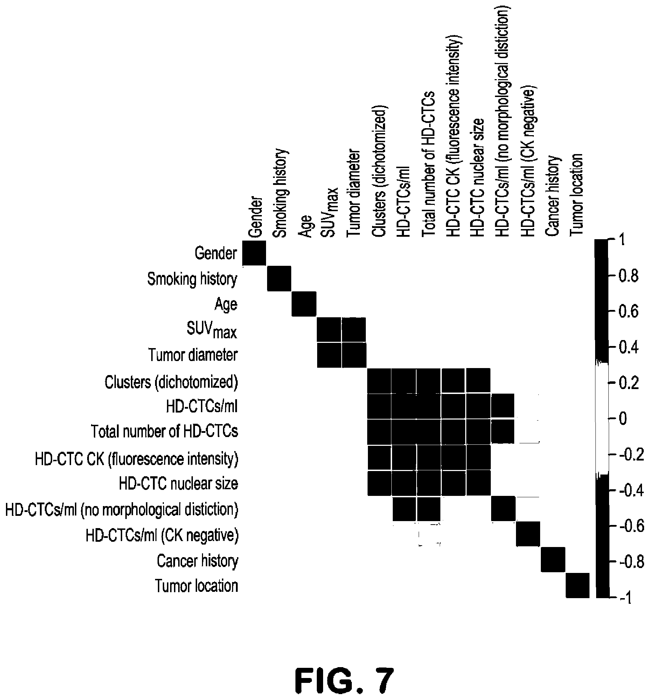

Figure 7. Variable correlations. Correlations for clinical (age, sex, smoking and cancer history), imaging (tumor diameter, location and SUVmax) and HD-CTC (including CTCs, clusters, DHCs, SHCs, CTC fluorescent intensity and CTC nuclear size) variables used for analysis are shown by hierarchical clustering. A correlation of 1 is perfect correlation and a correlation of 0 is no correlation at all. Correlations are symmetric around the diagonal of 1, which represents the correlation of a variable with itself. As shown, many of these features were not strongly correlated with each other, which explains their contribution to the LASSO model.)

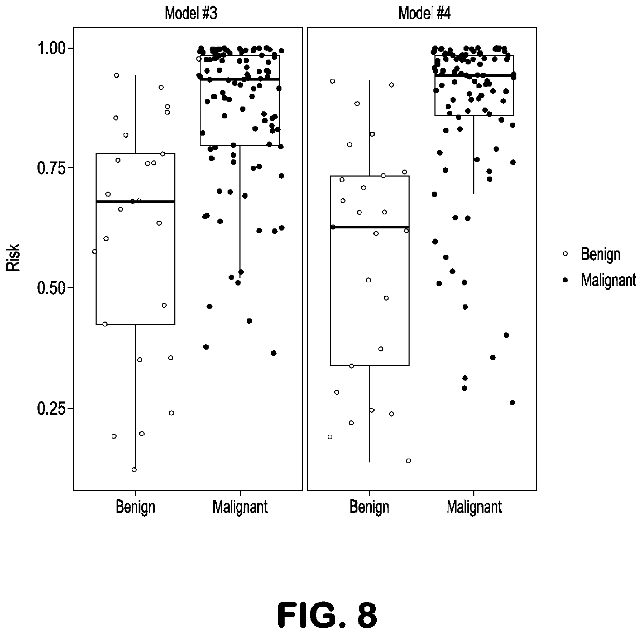

**Figure 8. Predicted cancer risk by disease group.** Risk scores calculated from regression modeling illustrate the high-risk nature of the benign cohort in comparison to the cases used for CTC analysis. Box plots are displayed with the median and interquartile range for predicted risk (y-axis) using models #2 and #4

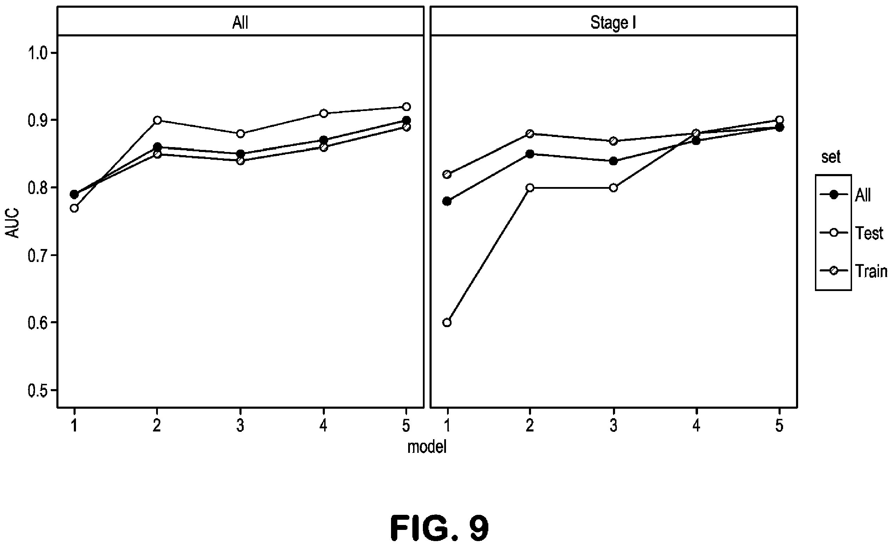

**Figure 9. AUC performance by model.** Models # 1-5 were analyzed for AUC test performance in a training and test set of patients. Note how the clinical model alone (Model #1) was inferior to the addition of HD-CTC data. Models # 4 and #5, both of which included HD-CTC clusters, performed best for all NSCLC and stage I disease alone.

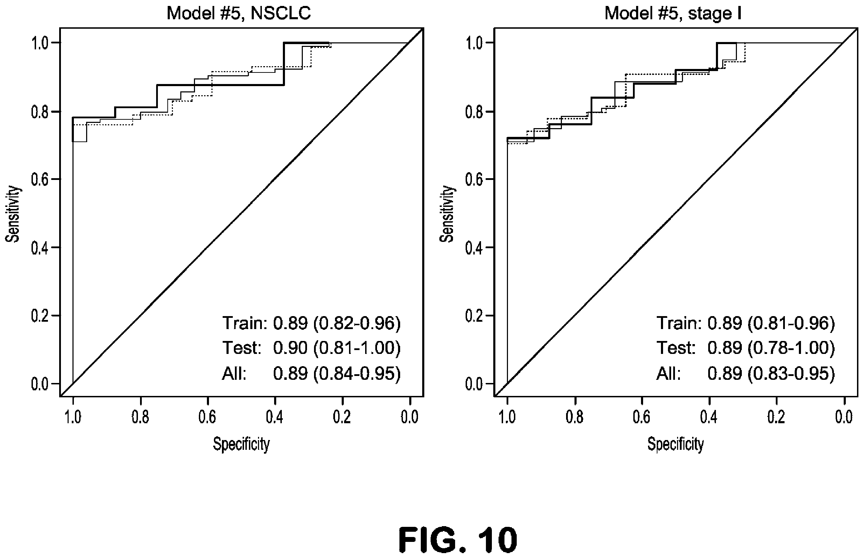

**Figure 10. Model #5 (LASSO Model).** AUCs Model #5 (LASSO Model) AUCs. Receiver operating characteristic (ROC) curves for the LASSO model for all NSCLC patients and by stage I disease only across training (dashed grey line), test (solid black line) and all (solid grey line) patients. AUCs for each cohort are shown in the lower right corner of each graph with 95% confidence intervals. The LASSO incorporated a combination of clinical, imaging and HD-CTC variables to yield the most discriminating model with consistency across cohorts.

## DETAILED DESCRIPTION

The present disclosure is based, in part, on the discovery that adding CTC data to existing clinical information enhances diagnostic accuracy for patients undergoing evaluation for lung cancer. As is described in detail below, the present disclosure demonstrates the integration of personal risk factors, imaging and CTC biomarkers to develop a risk score for predicting lung cancer in patients with NSCLC or stage I disease.

The present invention provides a method for diagnosing lung cancer in a subject comprising (a) generating circulating tumor cell (CTC) data from a blood sample obtained from the subject based on a direct analysis comprising immunofluorescent staining and morphological characteristics of nucleated cells in the sample, wherein CTCs are identified in context of surrounding nucleated cells based on a combination of the immunofluorescent staining and morphological characteristics (c) obtaining clinical data for the subject; (e) combining the CTC data with the clinical data to diagnose lung cancer in the subject.

The present invention also provides a method for diagnosing non-small cell lung cancer (NSCLC) in a subject comprising (a) generating circulating tumor cell (CTC) data from a blood sample obtained from the subject based on a direct analysis comprising immunofluorescent staining and morphological characteristics of nucleated cells in the sample, wherein CTCs are identified in context of surrounding nucleated cells based on a combination of the immunofluorescent staining and morphological characteristics (c) obtaining clinical data for the subject; (e) combining the CTC data with the clinical data to diagnose NSCLC in the subject.

The present invention also provides a method for diagnosing early stage NSCLC in a subject comprising (a) generating circulating tumor cell (CTC) data from a blood sample obtained from the subject based on a direct analysis comprising immunofluorescent staining and morphological characteristics of nucleated cells in the sample, wherein CTCs are identified in context of surrounding nucleated cells based on a combination of the immunofluorescent staining and morphological characteristics (c) obtaining clinical data for the subject; (e) combining the CTC data with the clinical data to diagnose early stage NSCLC in the subject.

It must be noted that, as used in this specification and the appended claims, the singular forms "a", "an" and "the" include plural referents unless the content clearly dictates otherwise. Thus, for example, reference to "a biomarker" includes a mixture of two or more biomarkers, and the like.

The term "about," particularly in reference to a given quantity, is meant to encompass deviations of plus or minus five percent.

As used in this application, including the appended claims, the singular forms "a," "an," and "the" include plural references, unless the content clearly dictates otherwise, and are used interchangeably with "at least one" and "one or more."

As used herein, the terms "comprises," "comprising," "includes," "including," "contains," "containing," and any variations thereof, are intended to cover a non-exclusive inclusion, such that a process, method, product-by-process, or composition of matter that comprises, includes, or contains an element or list of elements does not include only those elements but can include other elements not expressly listed or inherent to such process, method, product-by-process, or composition of matter.

The term "subject," as used herein includes humans as well as other mammals. It is noted that, as used herein, the terms "organism," "individual," "subject," or "patient" are used as synonyms and interchangeably.

As used herein, the term "circulating tumor cell" or "CTC" is meant to encompass any rare cell that is present in a biological sample that is related to lung cancer.

In its broadest sense, a biological sample can be any sample that contains CTCs. A sample can comprise a bodily fluid such as blood; the soluble fraction of a cell preparation, or an aliquot of media in which cells were grown; a chromosome, an organelle, or membrane isolated or extracted from a cell; genomic DNA, RNA, or cDNA in solution or bound to a substrate; a cell; a tissue; a tissue print; a fingerprint; cells; skin, and the like. A biological sample obtained from a subject can be any sample that contains cells and encompasses any material in which CTCs can be detected. A sample can be, for example, whole blood, plasma, saliva or other bodily fluid or tissue that contains cells.

In particular embodiments, the biological sample is a blood sample. As described herein, a preferred sample is whole blood, more preferably peripheral blood, still more preferably a peripheral blood cell fraction. As will be appreciated by those skilled in the art, a blood sample can include any fraction or component of blood, without limitation, T-cells, monocytes, neutrophiles, erythrocytes, platelets and microvesicles such as exosomes and exosome-like vesicles. In the context of this disclosure, blood cells included in a blood sample encompass any nucleated cells and are not limited to components of whole blood. As such, blood cells include, for example, both white blood cells (WBCs) as well as rare cells, including CTCs.

The samples of this disclosure can each contain a plurality of cell populations and cell subpopulation that are distinguishable by methods well known in the art (*e.g.,* FACS, immunohistochemistry). For example, a blood sample can contain populations of non-nucleated cells, such as erythrocytes (*e.g.*, 4-5 million/$\mu$l) or platelets (150,000-400,000 cells/$\mu$l), and populations of nucleated cells such as WBCs (*e.g.*, 4,500 - 10,000 cells/$\mu$l), CECs or CTCs (circulating tumor cells; *e.g.*, 2-800 cells/). WBCs may contain cellular subpopulations of, *e.g.*, neutrophils (2,500-8,000 cells/$\mu$l), lymphocytes (1,000-4,000 cells/$\mu$l), monocytes (100-700 cells/$\mu$l), eosinophils (50-500 cells/$\mu$l), basophils (25 - 100 cells/ $\mu$l) and the like. The samples of this disclosure are non-enriched samples, *i.e.*, they are not enriched for any specific population or subpopulation of nucleated cells. For example, non-enriched blood samples are not enriched for CTCs, WBC, B-cells, T-cells, NK-cells, monocytes, or the like.

In some embodiments the sample is a blood sample obtained from a healthy subject or a subject deemed to be at high risk of lung cancer based on art known clinically established criteria including, for example, smoking history and age. In some embodiments the blood sample is from a subject who has been diagnosed with NSCLC based on biopsy and/or surgery or clinical grounds. In some embodiments, the blood sample is obtained from a subject showing a clinical manifestation of NSCLC well known in the art or who presents with any of the known risk factors for NSCLC. The term "high risk" as used herein in the context of a subject's predisposition for NSCLC means current or recent smokers age 55 or older with a pack-year history of 30 years or more. As is understood by those skilled in the art, pack-year is a measure of how much an individual has smoked. For example, one pack-year of smoking corresponds to smoking one package of cigarettes (20 cigarettes) daily for one year.

As used herein in the context of generating CTC data, the term "direct analysis" means that the CTCs are detected in the context of all surrounding nucleated cells present in the sample as opposed to enrichment of the sample for CTCs prior to detection.

A fundamental aspect of the present disclosure is the robustness of the disclosed methods with regard to the detection of CTCs. The rare event detection (RED) disclosed herein with regard to CTCs is based on a direct analysis, *i.e.* non-enriched, of a population that encompasses the identification of rare events in the context of the surrounding non-rare events. Identification of the rare events according to the disclosed methods inherently identifies the surrounding events as non-rare events. Taking into account the surrounding non-rare events and determining the averages for non-rare events, for example, average cell size of non-rare events, allows for calibration of the detection method by removing noise. The result is a robustness of the disclosed methods that cannot be achieved with methods that are not based on direct analysis, but that instead compare enriched populations with inherently distorted contextual comparisons of rare events.

The disclosure provides methods for detecting CTCs in non-enriched blood samples and integrating CTC data with individual patient risk factors and imaging data to develop a risk score for predicting lung cancer in patients with NSCLC or stage I disease. The integration of CTC data with individual patient risk factors and imaging data significantly augments the use of individual patient risk factors and imaging data alone for risk stratifying patients undergoing an evaluation for lung cancer and provides a transformative non-invasive biomarker technology for diagnosing early stage non-small cell lung cancer (NSCLC). In some embodiments, the NSCLC is Stage I NSCLC.

As used herein, the term "clinical data" encompasses both lung imaging data and individual risk factors.

The term "imaging data" or "lung imaging data" as used herein, refers to any data generated via clinical imaging of a subject's lung and intergrated with other data to diagnose lung cancer, for example, early stage non-small cell lung cancer (NSCLC), in a subject according to the methods described herein. As such, the term includes data generated by any form of imaging modality known and used in the art, for example and without limitation, by chest X-ray and lung computed tomography (CT), lung ultrasound, positron emission tomography (PET), electrical impedance tomography and magnetic resonance (MRI). The term includes, for example and without limitation, maximum standard uptake value of the lesion ($SUV_{max}$), maximum nodule diameter and tumor location. It is understood that one skilled in the art can select lung imaging data based on a variety of art known criteria. As described herein, the methods of the invention can encompass one or more pieces of imaging data.

Lung imaging data can be generated through the use of any imaging modality known and used by those skilled in the art. Commonly used imaging modalities include chest radiograph, computed tomography (CT), scanning and/or magnetic resonance imaging (MRI), positron emission tomography (PET) scanning. In particular embodiments, the lung imaging data is generated comprising a positron emission tomography-computed tomography (PET/CT) scan. In further embodiements, the PET/CT is a 2-[18]-F-fluoro-2-deoxy-D-glucose (FDG) PET/CT (FDG PET/CT). While exemplified herein with *in-vivo* glycolytic marker FDG, any other marker can be selected by the skilled person to practice the invention methods.

As described herein, the clinical data generated and utilized in the methods of the invention can encompass one or more pieces of individual risk factors. As used herein, the term "individual risk factor" or "individual risk biomarker" refers to any measurable characteristic of a subject the change and/or the detection of which can be correlated with NSCLC and integrated with other data to diagnose lung cancer, for example, early stage NSCLC in the subject according to the methods described herein. In the methods disclosed herein, one or more individual risk factors can be selected from the group consisting of age, gender, ethnicity, cancer history, lung function and smoking status. It is understood that one skilled in the art can select additional individual risk factors based on a variety of art known criteria. As described herein, the methods of the invention can encompass one or more individual risk factors.

In the methods disclosed herein, CTC data and clinical data comprise measurable features. Measurable features useful for practicing the methods disclosed herein include any biomarker that can be correlated, individually or combined with other measurable features, with early stage non-small cell lung cancer (NSCLC) in a subject. Such biomarkers can include imaging data, individual risk factors and CTC data. CTC data can include both morphological features and immunofluorescent features. As will be understood by those skilled in the art, biomarkers can include a biological molecule, or a fragment of a biological molecule, the change and/or the detection of which can be correlated, individually or combined with other measurable features, with early stage non-small cell lung cancer (NSCLC) in a subject. Biomarkers also can include, but are not limited to, biological molecules comprising nucleotides, nucleic acids, nucleosides, amino acids, sugars, fatty acids, steroids, metabolites, peptides, polypeptides, proteins, carbohydrates, lipids, hormones, antibodies, regions of interest that serve as surrogates for biological macromolecules and combinations thereof (*e.g.*, glycoproteins, ribonucleoproteins, lipoproteins) as well as portions or fragments of a biological molecule.

CTCs, which can be present a single cells or in clusters of CTCs, are often epithelial cells shed from solid tumors and are present in very low concentrations in the circulation of subjects. Accordingly, detection of CTCs in a blood sample can be referred to as rare event detection. CTCs have an abundance of less than 1:1,000 in a blood cell population, *e.g.*, an abundance of less than 1:5,000, 1:10,000, 1:30,000, 1:50:000, 1:100,000, 1:300,000, 1:500,000, or 1:1,000,000. In some embodiments, the a CTC has an abundance of 1:50:000 to 1:100,000 in the cell population.

The samples of this disclosure may be obtained by any means, including, *e.g.*, by means of solid tissue biopsy or fluid biopsy (*see, e.g.,* Marrinucci D. et al., 2012, Phys. Biol. 9 016003). A blood sample may be extracted from any source known to include blood cells or components thereof, such as venous, arterial, peripheral, tissue, cord, and the like. The samples may be processed using well known and routine clinical methods (*e.g.*, procedures for drawing and processing whole blood). In some embodiments, a blood sample is drawn into anti-coagulent blood collection tubes (BCT), which may contain EDTA or Streck Cell-Free DNA™. In other embodiments, a blood sample is drawn into CellSave® tubes (Veridex). A blood sample may further be stored for up to 12 hours, 24 hours, 36 hours, 48 hours, or 60 hours before further processing.

In some embodiments, the methods of this disclosure comprise an intitial step of obtaining a white blood cell (WBC) count for the blood sample. In certain embodiments, the WBC count may be obtained by using a HemoCue® WBC device (Hemocue, Ängelholm, Sweden). In some embodiments, the WBC count is used to determine the amount of blood required to plate a consistent loading volume of nucleated cells per slide and to calculate back the equivalent of CTCs per blood volume.

In some embodiments, the methods of this disclosure comprise an initial step of lysing erythrocytes in the blood sample. In some embodiments, the erythrocytes are lysed, *e.g.,* by adding an ammonium chloride solution to the blood sample. In certain embodiments, a blood sample is subjected to centrifugation following erythrocyte lysis and nucleated cells are resuspended, *e.g.*, in a PBS solution.

In some embodiments, nucleated cells from a sample, such as a blood sample, are deposited as a monolayer on a planar support. The planar support may be of any material, *e.g.,* any fluorescently clear material, any material conducive to cell attachment, any material conducive to the easy removal of cell debris, any material having a thickness of < 100 $\mu$m. In some embodiments, the material is a film. In some embodiments the material is a glass slide. In certain embodiments, the method encompasses an initial step of depositing nucleated cells from the blood sample as a monolayer on a glass slide. The glass slide can be coated to allow maximal retention of live cells (*See, e.g.*, Marrinucci D. et al., 2012, Phys. Biol. 9 016003). In some embodiments, about 0.5 million, 1 million, 1.5 million, 2 million, 2.5 million, 3 million, 3.5 million, 4 million, 4.5 million, or 5 million nucleated cells are deposited onto the glass slide. In some embodiments, the methods of this disclosure comprise depositing about 3 million cells onto a glass slide. In additional embodiments, the methods of this disclosure comprise depositing between about 2 million and about 3 million cells onto said glass slide. In some embodiments, the glass slide and immobilized cellular samples are available for further processing or experimentation after the methods of this disclosure have been completed.

In some embodiments, the methods of this disclosure comprise an initial step of identifying nucleated cells in the non-enriched blood sample. In some embodiments, the nucleated cells are identified with a fluorescent stain. In certain embodiments, the fluorescent stain comprises a nucleic acid specific stain. In certain embodiments, the fluorescent stain is diamidino-2-phenylindole (DAPI). In some embodiments, immunofluorescent staining of nucleated cells comprises pan cytokeratin (CK), cluster of differentiation (CD) 45 and DAPI. In some embodiments further described herein, CTCs comprise distinct immunofluorescent staining from surrounding nucleated cells. In some embodiments, the distinct immunofluorescent staining of CTCs comprises DAPI (+), CK (+) and CD 45 (-). In some embbodiments, the identification of CTCs further comprises comparing the intensity of pan cytokeratin fluorescent staining to surrounding nucleated cells. In some embodiments, the CTC data is generated by fluorescent scanning microscopy to detect immunofluorescent staining of nucleated cells in a blood sample. Marrinucci D. et al., 2012, Phys. Biol. 9 016003).

CTCs, which can be present as single cells or in clusters of CTCs, are often epithelial cells shed from solid tumors found in very low concentrations in the circulation of patients. As used herein, the term "cluster" means two or more CTCs with touching cell membranes.

In particular embodiments, all nucleated cells are retained and immunofluorescently stained with monoclonal antibodies targeting cytokeratin (CK), an intermediate filament found exclusively in epithelial cells, a pan leukocyte specific antibody targeting the common leukocyte antigen CD45, and a nuclear stain, DAPI. The nucleated blood cells can be imaged in multiple fluorescent channels to produce high quality and high resolution digital images that retain fine cytologic details of nuclear contour and cytoplasmic distribution. While the surrounding WBCs can be identified with the pan leukocyte specific antibody targeting CD45, CTCs can be identified as DAPI (+), CK (+) and CD 45 (-). In the methods described herein, the CTCs comprise distinct immunofluorescent staining from surrounding nucleated cells.

In further embodiments, the CTC data includes high definition CTCs (HD-CTCs). HD-CTCs are CK positive, CD45 negative, contain an intact DAPI positive nucleus without identifiable apoptotic changes or a disrupted appearance, and are morphologically distinct from surrounding white blood cells (WBCs). DAPI (+), CK (+) and CD45 (-) intensities can be categorized as measurable features during HD-CTC enumeration as previously described (Figure 1). Nieva et al., Phys Biol 9:016004 (2012). The enrichment-free, direct analysis employed by the methods disclosed herein results in high sensitivity and high specificity, while adding high definition cytomorphology to enable detailed morphologic characterization of a CTC population known to be heterogeneous.

While CTCs can be identified as comprises DAPI (+), CK (+) and CD 45 (-) cells, the methods of the invention can be practiced with any other biomarkers that one of skill in the art selects for generating CTC data and/or identifying CTCs and CTC clusters. One skilled in the art knows how to select a morphological feature, biological molecule, or a fragment of a biological molecule, the change and/or the detection of which can be correlated with a CTC. Molecule biomarkers include, but are not limited to, biological molecules comprising nucleotides, nucleic acids, nucleosides, amino acids, sugars, fatty acids, steroids, metabolites, peptides, polypeptides, proteins, carbohydrates, lipids, hormones, antibodies, regions of interest that serve as surrogates for biological macromolecules and combinations thereof (e.g., glycoproteins, ribonucleoproteins, lipoproteins). The term also encompasses portions or fragments of a biological molecule, for example, peptide fragment of a protein or polypeptide

A person skilled in the art will appreciate that a number of methods can be used to generate CTC data, including microscopy based approaches, including fluorescence scanning microscopy (see, e.g., Marrinucci D. et al., 2012, Phys. Biol. 9 016003), mass spectrometry approaches, such as MS/MS, LC-MS/MS, multiple reaction monitoring (MRM) or SRM and product-ion monitoring (PIM) and also including antibody based methods such as immunofluorescence, immunohistochemistry, immunoassays such as Western blots, enzyme-linked immunosorbant assay (ELISA), immunopercipitation, radioimmunoassay, dot blotting, and FACS. Immunoassay techniques and protocols are generally known to those skilled in the art (Price and Newman, Principles and Practice of Immunoassay, 2nd Edition, Grove's Dictionaries, 1997; and Gosling, Immunoassays: A Practical Approach, Oxford University Press, 2000.) A variety of immunoassay techniques, including competitive and non-competitive immunoassays, can be used (Self et al., Curr. Opin. Biotechnol., 7:60-65 (1996), see also John R. Crowther, The ELISA Guidebook, 1st ed., Humana Press 2000, ISBN 0896037282 and, An Introduction to Radioimmunoassay and Related Techniques, by Chard T, ed., Elsevier Science 1995, ISBN 0444821198).

A person of skill in the art will futher appreciate that the presence or absence of biomarkers may be detected using any class of marker-specific binding reagents known in the art, including, e.g., antibodies, aptamers, fusion proteins, such as fusion proteins including protein receptor or protein ligand components, or biomarker-specific small molecule binders. In some embodiments, the presence or absence of CK or CD45 is determined by an antibody.

The antibodies of this disclosure bind specifically to a biomarker. The antibody can be prepared using any suitable methods known in the art. See, e.g., Coligan, Current Protocols in Immunology (1991); Harlow & Lane, Antibodies: A Laboratory Manual (1988); Goding, Monoclonal Antibodies: Principles and Practice (2d ed. 1986). The antibody can be any immunoglobulin or derivative thereof, whether natural or wholly or partially synthetically produced. All derivatives thereof which maintain specific binding ability are also included in the term. The antibody has a binding domain that is homologous or largely homologous to an immunoglobulin binding domain and can be derived from natural sources, or partly or wholly synthetically produced. The antibody can be a monoclonal or polyclonal antibody. In some embodiments, an antibody is a single chain antibody. Those of ordinary skill in the art will appreciate that antibody can be provided in any of a variety of forms including, for example, humanized, partially humanized, chimeric, chimeric humanized, *etc.* The antibody can be an antibody fragment including, but not limited to, Fab, Fab', F(ab')2, scFv, Fv, dsFv diabody, and Fd fragments. The antibody can be produced by any means. For example, the antibody can be enzymatically or chemically produced by fragmentation of an intact antibody and/or it can be recombinantly produced from a gene encoding the partial antibody sequence. The antibody can comprise a single chain antibody fragment. Alternatively or additionally, the antibody can comprise multiple chains which are linked together, for example, by disulfide linkages, and any functional fragments obtained from such molecules, wherein such fragments retain specific-binding properties of the parent antibody molecule. Because of their smaller size as functional components of the whole molecule, antibody fragments can offer advantages over intact antibodies for use in certain immunochemical techniques and experimental applications.

A detectable label can be used in the methods described herein for direct or indirect detection of the biomarkers when generating CTC data in the methods of the invention. A wide variety of detectable labels can be used, with the choice of label depending on the sensitivity required, ease of conjugation with the antibody, stability requirements, and available instrumentation and disposal provisions. Those skilled in the art are familiar with selection of a suitable detectable label based on the assay detection of the biomarkers in the methods of the invention. Suitable detectable labels include, but are not limited to, fluorescent dyes (*e.g.*, fluorescein, fluorescein isothiocyanate (FITC), Oregon Green™, rhodamine, Texas red, tetrarhodimine isothiocynate (TRITC), Cy3, Cy5, Alexa Fluor® 647, Alexa Fluor® 555, Alexa Fluor® 488), fluorescent markers (*e.g.*, green fluorescent protein (GFP), phycoerythrin, etc.), enzymes (*e.g.*, luciferase, horseradish peroxidase, alkaline phosphatase, etc.), nanoparticles, biotin, digoxigenin, metals, and the like.

For mass-sectrometry based analysis, differential tagging with isotopic reagents, *e.g.*, isotope-coded affinity tags (ICAT) or the more recent variation that uses isobaric tagging reagents, iTRAQ (Applied Biosystems, Foster City, Calif.), followed by multidimensional liquid chromatography (LC) and tandem mass spectrometry (MS/MS) analysis can provide a further methodology in practicing the methods of this disclosure.

A chemiluminescence assay using a chemiluminescent antibody can be used for sensitive, non-radioactive detection of proteins. An antibody labeled with fluorochrome also can be suitable. Examples of fluorochromes include, without limitation, DAPI, fluorescein, Hoechst 33258, R-phycocyanin, B-phycoerythrin, R-phycoerythrin, rhodamine, Texas red, and lissamine. Indirect labels include various enzymes well known in the art, such as horseradish peroxidase (HRP), alkaline phosphatase (AP), beta-galactosidase, urease, and the like. Detection systems using suitable substrates for horseradish-peroxidase, alkaline phosphatase, beta.-galactosidase are well known in the art.

A signal from the direct or indirect label can be analyzed, for example, using a microscope, such as a fluorescence microscope or a fluorescence scanning microscope. Alternatively, a spectrophotometer can be used to detect color from a chromogenic substrate; a radiation counter to detect radiation such as a gamma counter for detection of $^{125}$I; or a fluorometer to detect fluorescence in the presence of light of a certain wavelength. If desired, assays used to practice the methods of this disclosure can be automated or performed robotically, and the signal from multiple samples can be detected simultaneously.

In some embodiments, the biomarkers are immunofluorescent markers. In some embodiments, the immunofluorescent makers comprise a marker specific for epithelial cells In some embodiments, the immunofluorescent makers comprise a marker specific for white blood cells (WBCs). In some embodiments, one or more of the immunofluorescent markers comprise CD 45 and CK.

In some embodiments, the presence or absence of immunofluorescent markers in nucleated cells, such as CTCs or WBCs, results in distinct immunofluorescent staining patterns. Immunofluorescent staining patterns for CTCs and WBCs may differ based on which epithelial or WBC markers are detected in the respective cells. In some embodiments, determining presence or absence of one or more immunofluorescent markers comprises comparing the distinct immunofluorescent staining of CTCs with the distinct immunofluorescent staining of WBCs using, for example, immunofluorescent staining of CD45, which distinctly identifies WBCs. There are other detectable markers or combinations of detectable markers that bind to the various subpopulations of WBCs. These may be used in various combinations, including in combination with or as an alternative to immunofluorescent staining of CD45.

In some embodiments, CTCs comprise distinct morphological characteristics compared to surrounding nucleated cells. In some embodiments, the morphological characteristics comprise nucleus size, nucleus shape, cell size, cell shape, and/or nuclear to cytoplasmic ratio. In some embodiments, the method further comprises analyzing the nucleated cells by nuclear detail, nuclear contour, presence or absence of nucleoli, quality of cytoplasm, quantity of cytoplasm, intensity of immunofluorescent staining patterns. A person of ordinary skill in the art understands that the morphological characteristics of this disclosure may include any feature, property, characteristic, or aspect of a cell that can be determined and correlated with the detection of a CTC.

CTC data can be generated with any microscopic method known in the art. In some embodiments, the method is performed by fluorescent scanning microscopy. In certain embodiments the microscopic method provides high-resolution images of CTCs and their surrounding WBCs (*see, e.g.,* Marrinucci D. et al., 2012, Phys. Biol. 9 016003)). In some embodiments, a slide coated with a monolayer of nucleated cells from a sample, such as a non-enriched blood sample, is scanned by a fluorescent scanning microscope and the fluorescence intensities from immunofluorescent markers and nuclear stains are recorded to allow for the determination of the presence or absence of each immunofluorescent marker and the assessment of the morphology of the nucleated cells. In some embodiments, microscopic data collection and analysis is conducted in an automated manner.

In some embodiments, a CTC data includes detecting one or more biomarkers, for example, CK and CD 45. A biomarker is considered "present" in a cell if it is detectable above the background noise of the respective detection method used (*e.g.*, 2-fold, 3-fold, 5-fold, or 10-fold higher than the background; *e.g.*, $2\sigma$ or $3\sigma$ over background). In some embodiments, a biomarker is considered "absent" if it is not detectable above the background noise of the detection method used (*e.g.*, <1.5-fold or <2.0-fold higher than the background signal; *e.g.*, $<1.5\sigma$ or $<2.0\sigma$ over background).

In some embodiments, the presence or absence of immunofluorescent markers in nucleated cells is determined by selecting the exposure times during the fluorescence scanning process such that all immunofluorescent markers achieve a pre-set level of fluorescence on the WBCs in the field of view. Under these conditions, CTC-specific immunofluorescent markers, even though absent on WBCs are visible in the WBCs as background signals with fixed heights. Moreover, WBC-specific immunofluorescent markers that are absent on CTCs are visible in the CTCs as background signals with fixed heights. A cell is considered positive for an immunofluorescent marker (*i.e.,* the marker is considered present) if its fluorescent signal for the respective marker is significantly higher than the fixed background signal (*e.g.,* 2-fold, 3-fold, 5-fold, or 10-fold higher than the background; *e.g.*, $2\sigma$ or $3\sigma$ over background). For example, a nucleated cell is considered CD 45 positive (CD 45$^+$) if its fluorescent signal for CD 45 is significantly higher than the background signal. A cell is considered negative for an immunofluorescent marker (*i.e.,* the marker is considered absent) if the cell's fluorescence signal for the respective marker is not significantly above the background signal (*e.g.*, <1.5-fold or <2.0-fold higher than the background signal; *e.g.*, $<1.5\sigma$ or $<2.0\sigma$ over background).

Typically, each microscopic field contains both CTCs and WBCs. In certain embodiments, the microscopic field shows at least 1, 5, 10, 20, 50, or 100 CTCs. In certain embodiments, the microscopic field shows at least 10, 25, 50, 100, 250, 500, or 1,000 fold more WBCs than CTCs. In certain embodiments, the microscopic field comprises one or more CTCs or CTC clusters surrounded by at least 10, 50, 100, 150, 200, 250, 500, 1,000 or more WBCs.

In some embodiments of the methods for diagnosing, generation of the CTC data comprises enumeration of CTCs that are present in the blood sample. In some embodiments, a positive diagnosis of lung cancer comprises detection of at least 1.0 CTC/mL of blood, 1.5 CTCs/mL of blood, 2.0 CTCs/mL of blood, 2.5 CTCs/mL of blood, 3.0 CTCs/mL of blood, 3.5 CTCs/mL of blood, 4.0 CTCs/mL of blood, 4.5 CTCs/mL of blood, 5.0 CTCs/mL of blood, 5.5 CTCs/mL of blood, 6.0 CTCs/mL of blood, 6.5 CTCs/mL of blood, 7.0 CTCs/mL of blood, 7.5 CTCs/mL of blood, 8.0 CTCs/mL of blood, 8.5 CTCs/mL of blood, 9.0 CTCs/mL of blood, 9.5 CTCs/mL of blood, 10 CTCs/mL of blood, or more. In a particular embodiment, a positive diagnosis of lung cancer comprises detection of at least 7.5 CTC/mL of blood.

In some embodiments of the methods for diagnosing, generation of the CTC data comprises detecting CTC clusters. In some embodiments, a positive diagnosis of lung cancer comprises detection of at least 0.1 CTC cluster/mL of blood, 0.2 CTC clusters/mL of blood, 0.3 CTC clusters/mL of blood, 0.4 CTC clusters/mL of blood, 0.5 CTC clusters/mL of blood, 0.6 CTC clusters/mL of blood, 0.7 CTC clusters/mL of blood, 0.8 CTC clusters/mL of blood, 0.9 CTC clusters/mL of blood, 1 CTC cluster/mL of blood, 2 CTC clusters/mL of blood, 3 CTC clusters/mL of blood, 4 CTC clusters/mL of blood, 5 CTC clusters/mL of blood, 6 CTC clusters/mL of blood, 7 CTC clusters/mL of blood, 8 CTC clusters/mL of blood, 9 CTC clusters/mL of blood, 10 clusters/mL or more. In a particular embodiment, a positive diagnosis of lung cancer comprises detection of at least 1 CTC cluster/mL of blood.

In some embodiments, analyzing a measurable feature to determine the probability for lung cancer encompasses the use of a predictive model. In further embodiments, analyzing a measurable feature to determine the probability lung cancer in a subject encompasses comparing a measurable feature with a reference feature. As those skilled in the art can appreciate, such comparison can be a direct comparison to the reference feature or an indirect comparison where the reference feature has been incorporated into the predictive model. In further embodiments, analyzing a measurable feature to determine the probability the probability lung cancer in a subject encompasses one or more of a linear discriminant analysis model, a support vector machine classification algorithm, a recursive feature elimination model, a prediction analysis of microarray model, a logistic regression model, a CART algorithm, a flex tree algorithm, a LART algorithm, a random forest algorithm, a MART algorithm, a machine learning algorithm, a penalized regression method, or a combination thereof. In particular embodiments, the analysis comprises logistic regression. In additional embodiments, the diagnosis of lung cancer is expressed as a risk score.

An analytic classification process can use any one of a variety of statistical analytic methods to manipulate the quantitative data and provide for classification of the sample. Examples of useful methods include linear discriminant analysis, recursive feature elimination, a prediction analysis of microarray, a logistic regression, a CART algorithm, a FlexTree algorithm, a LART algorithm, a random forest algorithm, a MART algorithm, machine learning algorithms and other methods known to those skilled in the art.

Classification can be made according to predictive modeling methods that set a threshold for determining the

9

probability that a sample belongs to a given class. The probability preferably is at least 50%, or at least 60%, or at least 70%, or at least 80%, or at least 90% or higher. Classifications also can be made by determining whether a comparison between an obtained dataset and a reference dataset yields a statistically significant difference. If so, then the sample from which the dataset was obtained is classified as not belonging to the reference dataset class. Conversely, if such a comparison is not statistically significantly different from the reference dataset, then the sample from which the dataset was obtained is classified as belonging to the reference dataset class.

The predictive ability of a model can be evaluated according to its ability to provide a quality metric, e.g. AUROC (area under the ROC curve) or accuracy, of a particular value, or range of values. Area under the curve measures are useful for comparing the accuracy of a classifier across the complete data range. Classifiers with a greater AUC have a greater capacity to classify unknowns correctly between two groups of interest. ROC analysis can be used to select the optimal threshold under a variety of clinical circumstances, balancing the inherent tradeoffs that exist between specificity and sensitivity. In some embodiments, a desired quality threshold is a predictive model that will classify a sample with an accuracy of at least about 0.7, at least about 0.75, at least about 0.8, at least about 0.85, at least about 0.9, at least about 0.95, or higher. As an alternative measure, a desired quality threshold can refer to a predictive model that will classify a sample with an AUC of at least about 0.7, at least about 0.75, at least about 0.8, at least about 0.85, at least about 0.9, or higher. In some embodiments described herein, the method has a diagnostic accuracy comprising an AUC of at least about 0.80, 0.81, 0.82, 0.83, 0.84, 0.85, 0.86, 0.87, 0.88, 0.89, 0.90, or higher with a confidence interval of 0.82-0.94. In some embodiments, the AUC is at least about 0.80, 0.81, 0.82, 0.83, 0.84, 0.85, 0.86, 0.87, 0.88, 0.89, 0.90, or higher with a confidence interval of 0.94.

As is known in the art, the relative sensitivity and specificity of a predictive model can be adjusted to favor either the specificity metric or the sensitivity metric, where the two metrics have an inverse relationship. The limits in a model as described above can be adjusted to provide a selected sensitivity or specificity level, depending on the particular requirements of the test being performed. One or both of sensitivity and specificity can be at least about 0.7, at least about 0.75, at least about 0.8, at least about 0.85, at least about 0.9, or higher.

The raw data can be initially analyzed by measuring the values for each measurable feature or biomarker, usually in triplicate or in multiple triplicates. The data can be manipulated, for example, raw data can be transformed using standard curves, and the average of triplicate measurements used to calculate the average and standard deviation for each patient. These values can be transformed before being used in the models, e.g. log-transformed, Box-Cox transformed (Box and Cox, Royal Stat. Soc., Series B, 26:211-246(1964). The data are then input into a predictive model, which will classify the sample according to the state. The resulting information can be communicated to a patient or health care provider.

In some embodiments, the method disclosed herein for diagnosing early stage NSCLC in a subject has a specificity of >60%, >70%, >80%, >90% or higher. In additional embodiments, the method for diagnosing early stage NSCLC in a subject has a specificity >90% at a classification threshold of 7.5 CTCs/mL of blood. In additional embodiments, the method for diagnosing early stage NSCLC in a subject has a specificity at a classification threshold of one or more CTC clusters.

As will be understood by those skilled in the art, an analytic classification process can use any one of a variety of statistical analytic methods to manipulate the quantitative data and provide for classification of the sample. Examples of useful methods include, without limitation, linear discriminant analysis, recursive feature elimination, a prediction analysis of microarray, a logistic regression, a CART algorithm, a FlexTree algorithm, a LART algorithm, a random forest algorithm, a MART algorithm, and machine learning algorithms.

From the foregoing description, it will be apparent that variations and modifications can be made to the invention described herein to adopt it to various usages and conditions. Such embodiments are also within the scope of the following claims.

The recitation of a listing of elements in any definition of a variable herein includes definitions of that variable as any single element or combination (or subcombination) of listed elements. The recitation of an embodiment herein includes that embodiment as any single embodiment or in combination with any other embodiments or portions thereof.

All patents and publications mentioned in this specification are herein incorporated by reference to the same extent as if each independent patent and publication was specifically and individually indicated to be incorporated by reference.

The following examples are provided by way of illustration, not limitation.


**EXAMPLES**


Example 1. Integration of CTC Data with Clinical Information Enhances Diagnostic Accuracy for Patients Undergoing Evaluation for Lung Cancer

This Example confirms the utility CTCs as a viable diagnostic when added to integrated clinical and imaging data in early-stage disease, and further, developed a risk score for diagnosis of lung cancer.

This was a multicenter, prospective, observational study of CTCs in patients with a lung nodule or mass who were undergoing $^{18}$F-FDG PET-CT imaging for concern of lung cancer or staging of lung cancer by their referring physician. Patients were enrolled at three medical centers at or near to the time of PET-CT imaging after fully informed consent. Whole blood was collected through a peripheral, upper extremity vein after discarding the first one millileter to minimize skin tag contamination, and samples were shipped at ambient temperature and processed at The Scripps Research Institute (TSRI) within 48 hours. Prior to data analysis and integration of diagnosis with CTC data, the interpretation of the high-definition circulating tumor cell (HD-CTC) assay was performed without knowledge of the diagnosis in a single-blinded approach.

Cohort Development

Patients were divided into two separate cohorts using the first group of consecutively enrolled patients as a training group and the next group of consecutively enrolled patients as the test group. NSCLC was determined by biopsy and/or surgery (n = 102), or clinical grounds (n = 2 patients). Benignity was defined by the extracting physician (VSN, LB, JN) after reviewing the medical record and included patients who had surgically resected nodules that were not cancer (n = 7), a biopsy yielding an alternative diagnosis (n = 6), nodules that diminished over time with or without non-cancer related treatments (n = 7), or radiographic benign nodules per report (n = 5). Since the HD-CTC test is not specific to lung cancer alone, (Wendel et al., Phys Biol 9:016005, 2012; Marrinucci et al., Phys Biol 9:016003, 2012) any patients who had a competing diagnosis of another cancer, defined as undergoing evaluation or treatment for another cancer, were excluded to eliminate diagnostic confounding.

Cell features and enumerated thresholds for determining malignant from benign disease were generated to optimize the accuracy of the HD-CTC test alongside traditional clinical and imaging parameters of risk for a solitary pulmonary nodule. Schultz et al., Thorax 63:335-41, 2008. These variables were then carried forward to a test set of patients (n = 41) at the same medical centers, along with two additional medical centers with the same enrollment, phlebotomy, processing, and clinical extraction parameters to assess validity.

Research at all participating facilities was approved by their respective institutional review board. Previously published data exists for a subgroup of patients (n = 50) regarding CTC enumeration and its association with tumor FDG uptake. Nair et al., PLoS One 8:e67733, 2013

Data Extraction

Clinical data including age, gender, ethnicity, cancer history, and smoking status. A patient was defined as a current smoker if they were smoking at the time of enrollment, past smoker if they ever smoked and were not smoking at the time of enrollment, and non-smoker if they never smoked. Patients were followed over time through June 1, 2013 at all centers (12.3 months, IQR 3.7-16.7 months) and characterized as definitively malignant or benign, unknown, or lost to follow-up. Staging of cancer was defined according to the medical chart by the most recent TNM staging system (American Joint Committee on Cancer [AJCC] v 7.0). Rami-Porta et al., J Thorac Oncol 2:593-602, 2007. Imaging data collected included maximum standard uptake value of the lesion (SUV$_{max}$), maximum nodule diameter and tumor location. For lung region, upper and lower lung zones were analyzed; right middle lobe tumors were classified as lower lung zone tumors. No partial volume correction was performed for tumor SUV$_{max}$ in order to simulate the clinical setting. Shankar et al., J Nucl Med 47:1059-66, 2006

CTC enumeration

Sample evaluation for CTCs was performed as reported previously. Marrinucci et al. Phys Biol 9:016003, 2012 The technologist, microscopes and analysis systems were constant throughout the study. Approximately 10 million nucleated cells were assessed which represented approximately 1-2 mL of whole blood. Blood samples underwent hemolysis, centrifugation, re-suspension and plating onto custom adhesion slides (Marienfeld®, Lauda, Germany), followed by -80°C storage. Prior to analysis, slides were thawed, labeled by immunofluorescence (pan cytokeratin, CD45 and DAPI) and imaged by automated fluoroscopy then manual validation by a pathologist-trained technician (MSL). Marrinucci et al. Phys Biol 9:016003, 2012. DAPI (+), CK (+) and CD45 (-) intensities were categorized as features during HD-CTC enumeration as previously described (Figure 1). Nieva et al., Phys Biol 9:016004, 2012. Cells that only partially met these criteria were not deemed to be an HD-CTC by the technologist but were recorded as well. This included cells that were smaller than an accepted HD-CTC ("Small" HD-CTC Candidates or SHCs) or dimmer by CK staining than a HD-CTC ("Dim" HD-CTC Candidates or DHCs). Thus, the HD-CTC platform was able to categorize HD-CTC populations and unique "CTC like" candidate cells for analysis as previously described. Nieva et al., Phys Biol 9:016004, 2012. For cluster evaluation, groups of 2 HD-CTCs or more with touching cytoplasm as were defined as clusters.

Statistical Analysis

Summary statistics and frequencies were generated as appropriate. Continuous variables are reported as their median and interquartile range (IQR) for both parametric and non-parametric distributions. Differences between patients with NSCLC and benign nodules, as well as stage I disease only and benign lesions, were compared using a Student's t-test, Wilcoxon log-rank test, Chi-squared, Fisher exact test or Kruskal-Wallis test as appropriate and were annotated for a p-value < 0.05. For differences by histology, all non-adenocarcinomas were grouped together.

The variables included for modeling were i) clinical: age, sex, smoking, and cancer history; ii) FDG PET-CT derived: $SUV_{max}$, maximum lesion diameter and lesion location, iii) HD-CTC assay derived HD-CTC/mL, total HD-CTC clusters, and HD-CTC candidate cell features (SHCs and DHCs; Supplementary Figures 1 & 2). To analyze if HD-CTC assay derived features added value in addition to clinical and FDG PET-CT data (*model* #1), four multiple logistic regression models were calibrated using HD-CTC derived variables (*models* #2-5). Models #3-4 used HD-CTCs and clusters only along model#1 to assess diagnostic relevance while a LASSO approach (model#5) was used to agnostically select HD-CTC features and clinical variables. Tibshirani, J. Royal. Statist. Soc B 58:267-288, 1996. Those that added discriminating value to the logistic model at a p < 0.05 level (*i.e.*, having an odds ratio (OR) and 95% confidence interval [CI] that did not cross one) were considered statistically additive to the model. Next, receiver operating characteristic (ROC) curves were generated for illustrating the performance of each model for distinguishing benign patients from either all NSCLC patients or stage I patients only and report the area under the curve (AUC) and 95% confidence interval (CI). Sensitivity and specificity was reported in the training, test, and full data sets. Results were validated in all patients using 10 fold cross-validation (CV) to determine model stability. Picard and Cook, Journal of the American Statistical Association 79:575-583, 1984. This analysis was performed for all NSCLC cases and for stage I disease only vs. benign cases. Differences in AUCs were determined by comparing the CIs of the estimated AUCs generated from CV. We considered CIs that did not overlap between models a statistically significant result. Lastly, risk scores for the most significant models were developed using all patients with the coefficients (β) representing the contribution of the variable (x) to the risk model as follows:

$$y = \beta_{1 \ldots n} * (x)_{1 \ldots n} \qquad (1)$$

where the probability of cancer is equivalent to:

$$e^y / (1 + e^y) \qquad (2).$$

All analyses were performed using SAS EG (v 4.3; Cary, NC) and R (R v 3.0.1; Cary, NC, v 5.0). Ihaka and Gentleman, Journal of Computational and Graphical Statistics 5:299-314, 1996.

Using the methods described in the preceding paragraphs, a total of 170 patients were assessed, 117 in the training cohort and 53 in the test cohort (Figure 2). Ultimately, 129 patients (training = 88; test = 41) were eligible for further analysis following HD-CTC analysis, diagnostic verification, and elimination of confounding cancers. For all comers, median age was 69 years old (IQR 11), 84% of patients were current or past smokers and 63% of patients were male (Table 1). Overall lesion size for 104 NSCLCs and 25 benign lesions was 2.2 cm (IQR 1.4), $SUV_{max}$ was 4.5 (IQR 7.0). Eighty of the 104 NSCLCs were stage I, whose predominant histology was adenocarcinoma (68%). Significant differences existed between benign and malignant groups for age, tumor location, $SUV_{max}$, HD-CTC counts and HD-CTC clusters, while the training and test cohorts differed only in gender (Table 1). Notably, lesion size was not different between diagnostic groups but $SUV_{max}$ was.

Table 1. . Cohort Characteristics*

| | | All Patients | | | Training Cohort | Test Cohort |
|---|---|---|---|---|---|---|
| | | **All n=129** | **Benign n=25** | **Malignant n=104** | **All n=88** | **All n=41** |
| **CLINICAL MODEL** | Age (years) | 69±11 | 65±12[‡] | 69±11[‡] | 68±12 | 70±12 |
| | Gender (male) | 81 (63) | 17 (68) | 64 (62) | 63 (72)[‡] | 18 (44)[‡] |
| | Smoking history[†] | | | | | |
| | None | 21 (16) | 6 (24) | 15 (14) | 15 (17) | 6(15) |
| | Past | 78 (60) | 13 (52) | 64 (62) | 48 (55) | 29 (71) |
| | Current | 31 (24) | 6 (24) | 25 (24) | 25 (28) | 6 (15) |
| | Cancer history (yes) | 51 (40) | 13 (52) | 38 (37) | 38 (43) | 13 (32) |
| | Upper Lobe | 72 (56) | 10 (40)[‡] | 62 (60)[‡] | 50 (57) | 22 (54) |
| | Lesion size (cm)[§] | 2.2±1.4 | 2.1±2.3 | 2.2±1.3 | 2.3±1.8 | 2.3±1.4 |
| | SUV$_{max}$ | 4.5±7.0 | 2.6±2.1[‡] | 5.2±6.2[‡] | 4.0±7.0 | 5.3±5.7 |
| **CTC DATA** | Time to assay (hrs) | 24±2 | 23±4 | 24±2 | 24±3 | 24±2 |
| | mLs processed | 1.5±0.8 | 1.5±1.1 | 1.4±0.7 | 1.4±0.8 | 1.4±0.6 |
| | CTCs/mL[¶] | 3.6±15 | 0.7±4.0[‡] | 4.8±19[‡] | 3.7±11 | 3.7±10 |
| | Clusters (y/n) | 54 (42) | 2 (8)[‡] | 52 (50)[‡] | 35 (40) | 18 (44) |
| | Total clusters | 0±2 | 0±0[‡] | 1±3[‡] | 0±2 | 0±2 |

*Continuous variables shown with interquartile range (IQR, 25 to 75% range) for parametric and non-parametric variables. Differences between groups were tested using a Student's t-test or Wilcoxon log-rank test for parametric and non-parametric variables respectively. Categorical or ordinal variables were compared using Chi-squared, Fisher's exact test or Kruskal Wallis testing as appropriate. †Defined as current, ever or never per chart review. §Longest axis on PET-CT. ¶Standardized count by 10 million nucleated cells (WBC). ‡Significant differences by diagnosis at the $p < 0.05$ level.

A total of 4,291 HD-CTCs were discovered in malignant disease vs. 65 in benign disease (Figure 6). HD-CTC clusters ranged from 0 to 184 for malignant patients and 0 to 5 for benign lesions. HD-CTCs ranged from 0 to 378 in the malignant group (n=104) and from 0 to 21 in the benign group (n=25) (Figure 7). For stage I tumors, CTCs ranged from 0 to 297 (n=80). Forty-four patients of 104 patients (42%) had more than 7.5 HD-CTCs/mL for all NSCLC and 33/80 (41%) had more than 7.5 CTC/mL in the stage I only cohort. Fifty-two of 104 patients (50%) had HD-CTC clusters for all NSCLCs and 39/80 (49%) had HD-CTC clusters in stage I disease only. One patient had greater than 7.5 CTCs/mL (4%) in the benign group and two (8%) had at least one CTC cluster. There were no differences by histology (p = 0.22) or stage grouping (p = 0.39) for CTC counts.

Predicted risk from the logistic regression models for cancer in the benign group was comparable to the malignant group, confirming the high-risk nature of this cohort of patients regardless of disease state (Figure 8). As expected, and in-line with the extant literature (Schultz et al., Thorax 63:335-41, 2008), clinical data alone gave a reasonable accuracy for a diagnosis of any NSCLC vs. benign disease or for stage I vs. benign disease across cohorts (Table 2). Notably, age, maximal tumor diameter on CT, and tumor SUV$_{max}$ had the largest impact on the clinical model for all NSCLC patients and by stage I disease only (Supplemental Table 2 shown in Figure 5).

**Table 2.** Model Performance for Clinical Variables & CTC Features of Interest

| | | NSCLC vs. Benign | | | Stage I vs. Benign | | |
|---|---|---|---|---|---|---|---|
| | | *Training* *n = 88* | *Test* *n = 41* | *All* *n = 129* | *Training* *n = 71* | *Test* *n = 34* | *All* *n=105* |
| *Model #1* | AUC | 0.78 (0.68-0.89) | 0.75 (0.53-0.97) | 0.77 (0.68-0.87) | 0.80 (0.68-0.91) | 0.76 (0.54-0.97) | 0.79 (0.68-0.87) |
| *Clinical variables only*[*] | Sens | 0.65 | 0.91 | 0.71 | 0.83 | 0.85 | 0.68 |
| | Spec | 0.88 | 0.63 | 0.80 | 0.71 | 0.63 | 0.84 |
| *Model #2* | AUC | 0.84 (0.75-0.93) | 0.87 (0.73-1.00) | **0.86 (0.79-0.93)** | 0.88 (0.79-0.96) | 0.85 (0.70-1.00) | **0.86 (0.79-0.93)** |
| *With CTCs/mL*[§] | Sens | 0.84 | 0.84 | 0.74 | 0.69 | 0.80 | 0.75 |
| | Spec | 0.82 | 1.00 | 1.00 | 1.00 | 1.00 | 0.96 |
| *Model #3* | AUC | **0.84 (0.75-0.93)** | 0.83 (0.68-0.98) | **0.84 (0.77-0.92)** | **0.86 (0.77-0.96)** | 0.84 (0.70-0.99) | **0.86 (0.77-0.92)** |
| *With 7.5 CTCs/mL*[§] | Sens | 0.80 | 0.78 | 0.64 | 0.78 | 0.92 | 0.79 |
| | Spec | 0.82 | 1.00 | 0.96 | 0.88 | 0.63 | 0.80 |

| Model #4 | AUC | **0.88 (0.81-0.96)** | 0.88 (0.66-0.99) | **0.88 (0.82-0.94)** | **0.88 (0.79-0.97)** | 0.88 (0.75-1.00) | **0.87 (0.82-0.94)** |
|---|---|---|---|---|---|---|---|
| | Sens | 0.79 | 0.72 | 0.82 | 0.82 | 0.78 | 0.83 |
| *With CTC clusters* | | | | | | | |
| | Spec | 0.88 | 1.00 | 0.84 | 0.88 | 1.00 | 0.84 |
| *Model #5* | AUC | **0.89 (0.82-0.96)** | 0.90 (0.81-1.00) | **0.89 (0.84-0.95)** | **0.89 (0.81-0.96)** | 0.89 (0.78-1.00) | **0.89 (0.84-0.95)** |
| *LASSO model*[†§] | Sens | 0.72 | 0.78 | 0.83 | 0.82 | 0.64 | 0.81 |
| | Spec | 1.00 | 1.00 | 0.84 | 0.88 | 1.00 | 0.80 |

AUC = Area under curve (C-statistic) with 95% confidence interval. Sens = Sensitivity; Spec = Specificity. *See Table 1 for variables and levels included in the clinical model. †See methods. §In addition to model #1, see Supplemental Table 2 (Figure 5) for significant variables in each model. HD-CTC data that added value to the baseline clinical model at the p < 0.05 level are bolded.

HD-CTC/mL as a continuous variable yielded a marginal AUC (0.65) in the training cohort (Figure 3A) compared to the clinical model (AUC = 0.79), however, *in combination* with clinical data this biomarker was more discriminating (Table 2). When assessing whether an HD-CTC threshold improved accuracy for diagnosis, a threshold of 7.5 CTCs/mL was optimal and statistically added more value to logistic regression modeling in the training cohort (Table 2, Figure 3B). HD-CTC clusters were even more accurate than this dichotomized HD-CTCs/mL threshold for identifying disease state in the training model (Table 2; Figure 3C). Importantly, the LASSO approach (model#5) selected a combination of two clinical, three imaging and two HD-CTC features with the highest discrimination and confirmed that HD-CTC clusters added value to assigning a disease group (Table 2).

In general, specificity of the more accurate models was better than sensitivity (Table 2) and models #2-5 were better at identifying NSCLC than clinical models alone across cohorts (Figure 9). The most discriminating models from the training cohort performed well with similar AUCs in the test cohort (Table 2, Figure 3) but without statistical significance. Cross-validation showed that HD-CTC clusters significantly added information to the clinical model alone (model #4) in all patients with the LASSO (model #5) being the most significantly discriminating for all comers and for stage I disease only (Table 3; Figure 10). Using model #4's coefficients the following risk score using the variable levels defined in Table 1 was generated:

$$y = -6.79 + 0.106\ (Age) - 0.524\ (Gender) + 0.327\ (Smoking) - 0.375\ (Cancer\ history) - 1.05$$
$$(Location) + 0.184\ (SUV_{max}) + 2.54\ (HD\text{-}CTC\ clusters) \qquad (3)$$

**Table 3**. Cross-Validation of Models for NSCLC Patients and Stage I Disease

| Model | AUC (95% CI) |
|---|---|
| NSCLC Model #1, Clinical only | 0.72 (0.70–0.75) |
| NSCLC Model #2, Clinical and CTC/mL | 0.80 (0.77–0.82) |
| NSCLC Model #3, Clinical and 7.5 CTC/mL | 0.78 (0.75–0.81) |
| NSCLC Model #4, Clinical and HD-CTC clusters | **0.81 (0.77–0.84)** |
| NSCLC Model #5, LASSO Model | **0.84 (0.80–0.87)** |
| Stage I Model #1, Clinical only | 0.71 (0.68–0.74) |
| Stage I Model #2, Clinical and CTC/mL | 0.78 (0.75–0.82) |
| Stage I Model #3, Clinical and 7.5 CTC/mL | 0.78 (0.74–0.82) |
| Stage I Model #4, Clinical and HD-CTC clusters | **0.80 (0.76–0.83)** |
| Stage I Model #5, LASSO Model | **0.82 (0.79–0.85)** |

NSCLC = Non-small cell lung cancer. CI = Confidence interval. Bolded AUCs are significantly different from the baseline model

The results described herein, demonstrate the utility of the EpCAM independent HD-CTC platform in a highly relevant patient cohort with integrated clinical and molecular imaging data using an assay that utilizes blood at room temperature within 48 hours of phlebotomy. While many investigators have been interested in using CTCs as *prognostics* in cancer, including NSCLC, this study verified CTCs as a viable *diagnostic* when added to integrated clinical and imaging data in early-stage disease, and further, developed a risk score for diagnosis. To illustrate the utility of this score, the hypothetical example of a 71 year-old male smoker, with no cancer history and a 1.7 cm lower lobe nodule whose FDG PET-CT $SUV_{max}$ is 2.0 and whose blood reveals HD-CTC clusters is provided. Applying equation (3) and variable codes given in Supplemental Table 2 (shown in Figure 5), show that this patient would increase his pre-test probability of cancer from 53% to 94% with the addition of HD-CTC cluster data.

Although Tanaka *et al.* performed an important study using a similar patient cohort, they were unable to find a discriminating model using CTCs and did not integrate clinical or imaging data during analysis. Tanaka et al., Clin Cancer Res 15:6980-6, 2009. Their negative results may be in part be due to (1) sensitivity limitations of the CellSearch™ platform compared to the HD-CTC platform-since it is dependent on EpCAM antibody affinity-and/or (2) the lack of comparison to standard clinical variables of risk for identifying NSCLC patients, since orthogonally related biomarkers like FDG PET and CTCs appear to have additive value in the models. Nair et al., PLoS One 8:e67733, 2013.

The most discriminating models in the study included HD-CTC clusters. The rarity of such disease derived cell clusters, ranging from a few HD-CTCs aggregated together to "mega-clusters," and recent molecular characterizations describing their EMT phenotype suggest that clusters may be less likely inflammatory and a more "cancerous" subtype of putative CTCs. Brandt et al., Cancer Res 56:4556-61, 1996; Yu et al., Science 339:580-4, 2013; Liotta et al., Cancer Res 36:889-94, 1976. The data recapitulated this clinically since HD-CTC clusters showed the strongest diagnostic potential in the model, and suggest further patients should be studied to determine whether a clinical trial is indicated can ultimately bring this biomarker to the clinic in a short timeframe.

There is currently no gold standard for CTC detection and characterization that has diagnostic potential. While the original FDA approved CellSearch™ platform chose to use Epithelial Cell Adhesion Molecule (EpCAM) capture followed by CD45, CK and DAPI fluorescence to identify CTCs with claims of few false positives (Cristofanilli et al., N Engl J Med 351:781-91, 2004; Allard et al., Clin Cancer Res 10:6897-904, 2004), data published on the presence of CTCs in patients with benign disease undergoing a work-up for cancer in general, and lung cancer in particular, remain uncommon. Tanaka et al., Clin Cancer Res 15:6980-6, 2009; Pantel et al., Clin Chem 58:936-40, 2012. However, it was found that false positive results from the HD-CTC assay can arise from high-risk patients with other diseases including inflammation (Supplemental Table 1 shown in Figure 4). The data therefore suggest that, while enumeration appears to be clinically useful, even sensitive methods for detecting CTCs will benefit from additional molecular characterization to differentiate *circulating epithelial cells* (CECs) from *circulating tumor cells* (CTCs). Using additional immunofluorescent antibodies, next generation sequencing and/or single cell copy number variation analysis to define cells with pathognomonic hallmarks of cancer is one way to approach this issue.

This study has successfully married *in vitro* diagnostics (HD-CTCs) with *in vivo* molecular imaging data (*i.e.*, FDG-PET) and used a highly clinically relevant group of at-risk patients in a training-test blinded validation to develop a new risk score for predicting lung cancer. Key issues to address in the future relate to false positive HD-CTC results from lesions other than lung cancer that could be related either to 1) rare cells (*i.e.* CECs) from conditions such as infection or inflammation or 2) true positives (*i.e.* CTCs) in a pre-malignant patient. Case one seems supported by the current data for which follow-up blood draws will likely provide the answer. For case two, molecular phenotyping of HD-CTCs can provide definitive proof to differentiate CECs from CTCs.

## CONCLUSION

Putative CTCs detected using standard cell markers and cell morphology in the EpCAM independent HD-CTC platform were useful for risk stratifying patients undergoing an evaluation for lung cancer and augmented clinical models alone.

From the foregoing description, it will be apparent that variations and modifications can be made to the invention described herein to adopt it to various usages and conditions. Such embodiments are also within the scope of the following claims.

The recitation of a listing of elements in any definition of a variable herein includes definitions of that variable as any single element or combination (or subcombination) of listed elements. The recitation of an embodiment herein includes that embodiment as any single embodiment or in combination with any other embodiments or portions thereof.

All patents and publications mentioned in this specification are herein incorporated by reference to the same extent as if each independent patent and publication was specifically and individually indicated to be incorporated by reference.

Also disclosed herein are *inter alia* the following items:

1. A method for diagnosing lung cancer in a subject comprising

    (a) generating circulating tumor cell (CTC) data from a blood sample obtained from the subject based on a direct analysis comprising immunofluorescent staining and morphological characteristics of nucleated cells in said sample, wherein CTCs are identified in the context of surrounding nucleated cells based on a combination of said immunofluorescent staining and morphological characteristics;

    (b) obtaining clinical data for said subject;

    (c) combining said CTC data with said clinical data to diagnose lung cancer in said subject.

2. The method of item 1, wherein said clinical data comprises one or more pieces of imaging data.

3. The method of item 1, wherein said clinical data comprises one or more individual risk factors.

4. The method of item 1, wherein said lung cancer is non-small cell lung cancer (NSCLC).

5. The method of item 4, wherein said NSCLC is Stage I NSCLC.

6. The method of item 1, wherein the CTC data is generated by fluorescent scanning microscopy.

7. The method of item 6, wherein the CTC data is generated by assessing at least 4 million of said nucleated cells.

8. The method of item 6, wherein said microscopy provides a field of view comprising both CTCs and more than 200 surrounding white blood cells (WBCs).

9. The method of item 6, wherein said immunofluorescent staining of nucleated cells comprises pan cytokeratin, cluster of differentiation (CD) 45 and diamidino-2-phenylindole (DAPI).

10. The method of item 6, wherein said CTCs comprise distinct immunofluorescent staining from surrounding nucleated cells.

11. The method of item 10, wherein said distinct immunofluorescent staining comprises DAPI (+), CK (+) and CD 45 (-).

12. The method of item 1, wherein said CTCs comprise distinct morphological characteristics compared to surrounding nucleated cells.

13. The method of item 12, wherein said morphological characteristics comprise one or more of the group consisting of nucleus size, nucleus shape, cell size, cell shape and nuclear to cytoplasmic ratio.

14. The method of item 13, wherein said morphological characteristics further comprise one or more of the group consisting of nuclear detail, nuclear contour, presence or absence of nucleoli, quality of cytoplasm and quantity of cytoplasm.

15. The method of item 1, wherein said identification of CTCs further comprises comparing intensity of pan cytokeratin fluorescent staining to surrounding nucleated cells.

16. The method of item 1, further comprising an initial step of obtaining a white blood cell (WBC) count for the blood sample.

17. The method of item 1, further comprising an initial step of lysing erythrocytes in the blood sample.

18. The method of item 1, further comprising an initial step of depositing nucleated cells from the blood sample as a monolayer on a glass slide.

19. The method of item 18, further comprising depositing between about 2 million and about 3 million cells onto said glass slide.

20. The method of item 1, wherein the generation of said CTC data comprises enumeration of CTCs in the blood sample.

21. The method of item 20, wherein a positive diagnosis of lung cancer comprises detection of at least 7.5 CTCs/mL of blood.

22. The method of item 20, wherein the generation of said CTC data comprises detecting CTC clusters.

23. The method of item 22, wherein a positive diagnosis of lung cancer comprises detection of one or more CTC clusters.

24. The method of item 2, wherein said imaging data is generated comprising a positron emission tomography-computed tomography (PET/CT) scan.

25. The method of item 17, wherein said PET/CT is a 2-[18]-F-fluoro-2-deoxy-D-glucose (FDG) PET/CT (FDG PET/CT).

26. The method of item 25, wherein said one or more pieces of imaging data are selected from the group consisting of maximum standardized uptake value ($SUV_{max}$), maximum lesion diameter and lesion location.

27. The method of item 2, wherein said one or more individual risk factors are selected from the group consisting of age, gender, ethnicity, cancer history, and smoking status.

28. The method of item 1, wherein said CTC data and said clinical data comprise measurable features.

29. The method of item 28, wherein said measurable features are analyzed using a predictive model.

30. The method of item 29, wherein said analysis comprises logistic regression.

31. The method of item 30, wherein the diagnosis is expressed as a risk score.

32. The method of item 30, wherein said method has a diagnostic accuracy comprising an area under the ROC curve (AUROC) of at least about 0.88.

33. The method of item 32, wherein the AUROC is at least 0.94.

34. The method of item 1, wherein said subject is a high risk subject.

**Claims**

1. A method for diagnosing lung cancer in a subject, the method comprising:

   (a) generating circulating tumor cell (CTC) data from a blood sample obtained from the subject based on a direct analysis of CTCs detected in the context of surrounding nucleated cells present in the sample in the absence of enrichment of the CTCs prior to detection, comprising immunofluorescent staining and morphological characteristics of nucleated cells in said sample, wherein CTCs are identified in the context of surrounding nucleated cells based on a combination of said immunofluorescent staining and morphological characteristics;
   (b) obtaining clinical data for said subject; and
   (c) combining said CTC data with said clinical data to diagnose lung cancer in said subj ect.

2. The method of claim 1, wherein said clinical data comprises one or more pieces of imaging data, optionally wherein said imaging data is generated comprising a positron emission tomography-computed tomography (PET/CT) scan, optionally wherein said PET/CT is a 2-[18]-F-fluoro-2-deoxy-D-glucose (FDG) PET/CT (FDG PET/CT), optionally wherein said one or more pieces of imaging data are selected from maximum standardized uptake value (SUVmax), maximum lesion diameter and lesion location.

3. The method of claim 1, wherein said clinical data comprises one or more individual risk factors, optionally wherein said one or more individual risk factors are selected from age, gender, ethnicity, cancer history, and smoking status.

4. The method of claim 1, wherein said lung cancer is non-small cell lung cancer (NSCLC), optionally wherein said NSCLC is Stage I NSCLC.

5. The method of claim 1, wherein the CTC data is generated by fluorescent scanning microscopy.

6. The method of claim 5, wherein the CTC data is generated by assessing at least 4 million of said nucleated cells; or wherein said microscopy provides a field of view comprising both CTCs and more than 200 surrounding white blood cells (WBCs).

7. The method of claim 5, wherein said immunofluorescent staining of nucleated cells comprises pan cytokeratin, cluster of differentiation (CD) 45 and diamidino-2-phenylindole (DAPI); or wherein said CTCs comprise distinct immunofluorescent staining from surrounding nucleated cells, optionally wherein said distinct immunofluorescent staining comprises DAPI (+), CK (+) and CD 45 (-) .

8. The method of claim 1, wherein said CTCs comprise distinct morphological characteristics compared to surrounding nucleated cells, optionally wherein said morphological characteristics comprise one or more of the group consisting of nucleus size, nucleus shape, cell size, cell shape and nuclear to cytoplasmic ratio, optionally wherein said morphological characteristics further comprise one or more of nuclear detail, nuclear contour, presence or absence of nucleoli, quality of cytoplasm and quantity of cytoplasm.

9. The method of claim 1, wherein said identification of CTCs further comprises comparing intensity of pan cytokeratin fluorescent staining to surrounding nucleated cells.

10. The method of claim 1, further comprising an initial step of obtaining a white blood cell (WBC) count for the blood sample; or further comprising an initial step of lysing erythrocytes in the blood sample.

11. The method of claim 1, further comprising an initial step of depositing nucleated cells from the blood sample as a monolayer on a glass slide;
optionally depositing between about 2 million and about 3 million cells onto said glass slide.

12. The method of claim 1, wherein the generation of said CTC data comprises enumeration of CTCs in the blood sample, optionally wherein:

a positive diagnosis of lung cancer comprises detection of at least 7.5 CTCs/mL of blood; or
the generation of said CTC data comprises detecting CTC clusters, optionally wherein a positive diagnosis of lung cancer comprises detection of one or more CTC clusters.

13. The method of claim 1, wherein said CTC data and said clinical data comprise measurable features,
optionally wherein said measurable features are analyzed using a predictive model.

14. The method of claim 13, wherein said analysis comprises logistic regression, optionally wherein:

the diagnosis is expressed as a risk score; or
said method has a diagnostic accuracy comprising an area under the ROC curve (AUROC) of at least about 0.88, preferably wherein the AUROC is at least 0.94.

15. The method of claim 1, wherein said subject is a high risk subject.

**Figure 1**

**Total, n = 170**

Training Cohort

Test Cohort

n = 117

Process Failure (9)
-1 Sample not processed
-4 Samples processed incorrectly
-4 Samples insufficient material

Process Failure (4)
-1 Sample not processed
-1 Sample processed > 48 hours
-2 Samples insufficient material

n = 53

n = 108

Diagnosis (20)
-11 Non NSCLC
-9 Competing cancer

Diagnosis (8)
-4 Unclear diagnosis
-1 Sample with synchronous primaries
-1 Sample with no nodule
-2 nodules no PET

n = 49

n = 88

Stage II-IV NSCLC (17)

Analysis I:
NSCLC vs Benign

Stage II-IV NSCLC (7)

n = 41

**n = 71**

Analysis II:
Stage vs Benign

n = 34

**Stage I, n = 54**
**Benign, n = 17**

**Stage I, n = 26**
**Benign, n = 8**

# FIG. 2

**FIG. 3**

**Supplemental Table 1:** Analysis of 25 benign patients with circulating epithelial cells using the HD-CTC assay

| Total CTC | CTC/mL | Clusters | Cohort | Center | Age (yrs) | Gender | Smoking History | Cancer History | Lesion Location | SUV max | Lesion Diameter (cm) | TTA (hrs) | mL/ test | Description |
|---|---|---|---|---|---|---|---|---|---|---|---|---|---|---|
| 16 | 14.9 | 2 | Training | PAVAHCS | 65 | Male | past | Yes | RML | 0.7 | 1 | 25 | 1.11 | Intra-pulmonary lymph node by surgical resection |
| 8 | 4.7 | 0 | Training | Stanford | 65 | Male | current | No | RML | 12.3 | 2.2 | 21 | 2.21 | Video Assisted Thoracoscopy showed NTMB |
| 7 | 4.0 | 0 | Training | Stanford | 62 | Female | past | No | LUL | 1.7 | 1.3 | 25 | 1.21 | Stable nodule over five years when compared to previous chest x-ray |
| 5 | 4.4 | 0 | Training | Stanford | 71 | Male | past | No | LUL | 2.4 | 2.4 | 19 | 1.52 | Resolution of nodule on follow up imaging |
| 5 | 3.2 | 0 | Test | CPMC | 43 | Female | none | Yes | RUL | 9.9 | 4.5 | 18 | 0.97 | M. *tuberculosis* on biopsy |
| 5 | 4.8 | 0 | Training | Stanford | 69 | Male | past | No | RUL | 2.6 | 1.9 | 23 | 2.06 | C. *immitis* fungal nodule, resected surgically |
| 4 | 4.8 | 0 | Test | Stanford | 77 | Male | none | Yes | LLL | 1.5 | 1.9 | 25 | 2.13 | Stability of round atelectasis over 2 years |
| 4 | 3.7 | 0 | Test | PAVAHCS | 69 | Male | past | Yes | LUL | 2.7 | 5.4 | 27 | 1.35 | Resolution of nodule on follow-up imaging |
| 3 | 3.1 | 0 | Training | PAVAHCS | 58 | Female | current | Yes | RUL | 0.38 | 1 | 23 | 0.88 | Follow-up chest x-ray shows no evidence of lesion |
| 2 | 0.9 | 0 | Training | Stanford | 63 | Female | none | Yes | RLL | 6.9 | 2.3 | 28 | 2.38 | NTMB resected surgically |
| 2 | 0.4 | 0 | Training | Stanford | 75 | Male | past | No | RUL | 1.6 | 2.1 | 21 | 1.11 | Biopsy proven granuloma and necrosis question of NTMB disease |
| 1 | 0.5 | 0 | Training | Stanford | 70 | Male | current | No | RLL | 9 | 3.8 | 24 | 2.70 | Resolution of round pneumonia on follow up imaging |
| 1 | 2.8 | 0 | Training | Stanford | 72 | Male | past | Yes | RLL | 1.8 | 2 | 22 | 1.33 | Lung abscess by biopsy |
| 1 | 0.7 | 1 | Training | Stanford | 26 | Female | none | No | LUL | 2 | 1 | 19 | 1.77 | Decreasing size of nodule with anti-bacterial therapy |
| 1 | 1.0 | 0 | Training | PAVAHCS | 55 | Male | current | No | RLL | 0.5 | 1.2 | 21 | 0.70 | Benign per PET read (calcified with smooth margins) |
| 0 | 0.0 | 0 | Training | PAVAHCS | 67 | Male | none | No | RLL | 1.4 | 1.6 | 24 | 1.09 | Decreasing size of nodule over time |
| 0 | 0.0 | 0 | Test | CPMC | 50 | Female | past | Yes | RLL | 0.9 | 0.8 | 23 | 2.47 | Hamartoma by surgical resection |
| 0 | 0.0 | 0 | Training | PAVAHCS | 51 | Male | current | Yes | RLL | 11 | 6 | 24 | 4.23 | Lung abscess by surgical resection |
| 0 | 0.0 | 0 | Test | PAVAHCS | 60 | Male | past | No | LUL | 3 | 4 | 24 | 0.90 | Resolution of lesion on follow-up imaging |
| 0 | 0.0 | 0 | Training | PAVAHCS | 61 | Male | current | Yes | RLL | 3.3 | 4.5 | 21 | 1.01 | Round atelectasis by imaging and superimposed Nocardia infection |
| 0 | 0.0 | 0 | Test | Stanford | 60 | Female | past | No | RLL | 7.6 | 3.9 | 22 | 2.28 | Allergic bronchopulmonary aspergillosis with fleeting nodules |
| 0 | 0.0 | 0 | Training | Stanford | 53 | Male | none | No | LUL | 0.7 | 1.7 | 21 | 1.51 | C. *immitis* fungal nodule on biopsy |
| 0 | 0.0 | 0 | Test | CPMC | 68 | Female | past | Yes | LLL | 3.1 | 1.6 | 27 | 1.89 | Granulomataous disease (possibly H. *capsulatum*) by surgical resection |
| 0 | 0.0 | 0 | Training | PAVAHCS | 78 | Male | past | Yes | RLL | 3.1 | 4 | 24 | 2.38 | C. *immitis* pneumonia by biopsy |
| 0 | 0.0 | 0 | Test | Billings | 80 | Male | past | Yes | LLL | 3.6 | 2.2 | 27 | 1.37 | Mediastinoscopy positive for H. *capsulatum* |

# FIG. 4

**Supplemental Table 2:** Logistic regression coefficients by model

Table 1:

| | Dependent variable: | | | | |
| --- | --- | --- | --- | --- | --- |
| | (1) | (2) | (3) | (4) | (5) |
| Age (years) | 0.091*** | 0.095*** | 0.104*** | 0.104*** | 0.104*** |
| | (0.029) | (0.032) | (0.033) | (0.030) | (0.032) |
| Gender (male) | -0.413 | -0.050 | -0.216 | -0.188 | -0.313 |
| | (0.621) | (0.675) | (0.665) | (0.689) | (0.676) |
| Smoking history (current/past/no) | 0.140 | 0.438 | 0.533 | 0.517 | |
| | (0.403) | (0.426) | (0.433) | (0.432) | |
| Cancer history (yes) | -0.248 | -0.610 | -0.694 | -0.441 | |
| | (0.530) | (0.587) | (0.588) | (0.590) | |
| SUV$_{max}$ | 0.210** | 0.186** | 0.158** | 0.179** | 0.202** |
| | (0.089) | (0.088) | (0.086) | (0.080) | (0.085) |
| Tumor diameter (cm) | -0.460** | -0.612** | -0.532** | -0.431** | -0.440 |
| | (0.216) | (0.279) | (0.239) | (0.250) | (0.287) |
| Tumor location (upper/lower) | 0.264 | 0.654 | 0.571 | 0.682 | 0.571 |
| | (0.530) | (0.596) | (0.593) | (0.612) | (0.617) |
| HD-CTC/ml | | 0.125** | | | |
| | | (0.063) | | | |
| HD-CTC (dichotomized) | | | 2.745*** | | |
| | | | (0.966) | | |
| HD-CTC canadidates/ml (no morphologic distinction) | | | | | 0.099* |
| | | | | | (0.052) |
| HD-CTC Clusters (dichotomized) | | | | 2.965*** | 2.537*** |
| | | | | (0.932) | (0.934) |
| Constant | -4.456** | -5.662** | -5.998** | -6.608*** | -6.875*** |
| | (1.968) | (2.291) | (2.300) | (2.167) | (2.261) |
| Log Likelihood | -49.785 | -41.872 | -43.418 | -41.300 | -39.062 |
| Akaike Inf. Crit. | 115.569 | 101.743 | 104.836 | 100.600 | 94.124 |

*Note:* *p<0.1; **p<0.05; ***p<0.01

# FIG. 5

**FIG. 6**

FIG. 7

FIG. 8

**FIG. 9**

FIG. 10

EP 3 702 785 A1

Europäisches
Patentamt

European
Patent Office

Office européen
des brevets

**EUROPEAN SEARCH REPORT**

Application Number

EP 20 15 6084

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | MARRINUCCI DENA ET AL: "Circulating tumor cells from well-differentiated lung adenocarcinoma retain cytomorphologic features of primary tumor type", STEROID PROFILES USING LIQUID CHROMATOGRAPHY-TANDEM MASS SPECTROMETRY WITH ATMOSPHERIC PRESSURE PHOTOIONIZATION SOURCE,, vol. 133, no. 9, 1 September 2009 (2009-09-01), pages 1468-1471, XP009194183, ISSN: 1543-2165, DOI: 10.1043/1543-2165-133.9.1468 * the whole document * | 1-15 | INV. G01N33/574 G01N21/00 |
| A | TUFMAN AMANDA ET AL: "Biological markers in lung cancer: A clinician's perspective", CANCER BIOMARKERS : SECTION A OF DISEASE MARKERS, IOS PRESS, NL, vol. 6, no. 3-4, 1 January 2009 (2009-01-01), pages 123-135, XP009171625, ISSN: 1875-8592, DOI: 10.3233/CBM-2009-0124 [retrieved on 2010-07-26] * the whole document * | 1-15 | |

TECHNICAL FIELDS
SEARCHED      (IPC)

G01N

-/--

The present search report has been drawn up for all claims

| Place of search | Date of completion of the search | Examiner |
|---|---|---|
| Munich | 7 May 2020 | Moreno de Vega, C |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

page 1 of 3

Europäisches Patentamt

European Patent Office

Office européen des brevets

**EUROPEAN SEARCH REPORT**

Application Number

EP 20 15 6084

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | MATTHEW G. KREBS ET AL: "Analysis of Circulating Tumor Cells in Patients with Non-small Cell Lung Cancer Using Epithelial Marker-Dependent and -Independent Approaches", JOURNAL OF THORACIC ONCOLOGY, vol. 7, no. 2, 1 February 2012 (2012-02-01), pages 306-315, XP055367672, Philadelphia, PA, US ISSN: 1556-0864, DOI: 10.1097/JTO.0b013e31823c5c16 * the whole document * | 1-15 | |
| X | MARCO WENDEL ET AL: "Fluid biopsy for circulating tumor cell identification in patients with early-and late-stage non-small cell lung cancer: a glimpse into lung cancer biology.", PHYSICAL BIOLOGY, INSTITUTE OF PHYSICS PUBLISHING, BRISTOL, GB, vol. 9, no. 1, 3 February 2012 (2012-02-03), page 16005, XP020218198, ISSN: 1478-3975, DOI: 10.1088/1478-3967/9/1/016005 | 1-4,6-15 | |
| | | | **TECHNICAL FIELDS SEARCHED (IPC)** |
| Y | * the whole document * | 1-15 | |
| X | VISWAM S. NAIR ET AL: "An Observational Study of Circulating Tumor Cells and 18F-FDG PET Uptake in Patients with Treatment-Naive Non-Small Cell Lung Cancer", PLOS ONE, vol. 8, no. 7, 1 July 2013 (2013-07-01), pages 1-9, XP055355514, DOI: 10.1371/journal.pone.0067733 * the whole document * | 1-15 | |

-/--

The present search report has been drawn up for all claims

| Place of search | Date of completion of the search | Examiner |
|---|---|---|
| Munich | 7 May 2020 | Moreno de Vega, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 3

Europäisches
Patentamt

European
Patent Office

Office européen
des brevets

**EUROPEAN SEARCH REPORT**

Application Number

EP 20 15 6084

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | LUTTGEN MADELYN S ET AL: "Developing a non-invasive, diagnostic test for stage I non-small cell lung cancer using circulating tumor cells", CANCER RESEARCH, AMERICAN ASSOCIATION FOR CANCER RESEARCH, US, vol. 73, no. 8, suppl. 1, 1 April 2013 (2013-04-01), XP009194211, ISSN: 0008-5472, DOI: 10.1158/1538-7445.AM2013-3485 * the whole document * ----- | 1-15 | |
| | | | TECHNICAL FIELDS SEARCHED (IPC) |

The present search report has been drawn up for all claims

| Place of search | Date of completion of the search | Examiner |
|---|---|---|
| Munich | 7 May 2020 | Moreno de Vega, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 61921694

**Non-patent literature cited in the description**

- **CRISTOFANILLI et al.** *N Engl J Med,* 2004, vol. 351, 781-91
- **MARRINUCCI D. et al.** *Phys. Biol.,* 2012, vol. 9, 016003
- **NIEVA et al.** *Phys Biol,* 2012, vol. 9, 016004
- **PRICE ; NEWMAN.** Principles and Practice of Immunoassay. Grove's Dictionaries, 1997
- **GOSLING.** Immunoassays: A Practical Approach. Oxford University Press, 2000
- **SELF et al.** *Curr. Opin. Biotechnol.,* 1996, vol. 7, 60-65
- **JOHN R. CROWTHER.** The ELISA Guidebook. Humana Press, 2000
- An Introduction to Radioimmunoassay and Related Techniques. Elsevier Science, 1995
- **COLIGAN.** *Current Protocols in Immunology,* 1991
- **HARLOW ; LANE.** Antibodies: A Laboratory Manual. 1988
- **GODING.** Monoclonal Antibodies: Principles and Practice. 1986
- **BOX ; COX.** *Royal Stat. Soc.,* 1964, vol. 26, 211-246
- **WENDEL et al.** *Phys Biol,* 2012, vol. 9, 016005
- **MARRINUCCI et al.** *Phys Biol,* 2012, vol. 9, 016003
- **SCHULTZ et al.** *Thorax,* 2008, vol. 63, 335-41
- **NAIR et al.** *PLoS One,* 2013, vol. 8, e67733
- **RAMI-PORTA et al.** *J Thorac Oncol,* 2007, vol. 2, 593-602
- **SHANKAR et al.** *J Nucl Med,* 2006, vol. 47, 1059-66
- **TIBSHIRANI ; J. ROYAL.** *Statist. Soc B,* 1996, vol. 58, 267-288
- **PICARD ; COOK.** *Journal of the American Statistical Association,* 1984, vol. 79, 575-583
- **IHAKA ; GENTLEMAN.** *Journal of Computational and Graphical Statistics,* 1996, vol. 5, 299-314
- **TANAKA et al.** *Clin Cancer Res,* 2009, vol. 15, 6980-6
- **BRANDT et al.** *Cancer Res,* 1996, vol. 56, 4556-61
- **YU et al.** *Science,* 2013, vol. 339, 580-4
- **LIOTTA et al.** *Cancer Res,* 1976, vol. 36, 889-94
- **ALLARD et al.** *Clin Cancer Res,* 2004, vol. 10, 6897-904
- **PANTEL et al.** *Clin Chem,* 2012, vol. 58, 936-40